(19) Europäisches Patentamt
European Patent Office
Office européen des brevets

(11) **EP 4 651 146 A1**

(12) **EUROPEAN PATENT APPLICATION**

(43) Date of publication:
**19.11.2025 Bulletin 2025/47**

(21) Application number: **25175433.9**

(22) Date of filing: **09.05.2025**

(51) International Patent Classification (IPC):
**G16H 20/17** (2018.01)  **A61B 5/00** (2006.01)
**G16H 50/20** (2018.01)  **G16H 50/30** (2018.01)
**G16H 50/70** (2018.01)

(52) Cooperative Patent Classification (CPC):
**G16H 20/17; A61B 5/14532; A61B 5/4839;
G16H 50/20; G16H 50/30; G16H 50/70**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **16.05.2024 US 202463648352 P
30.04.2025 US 202519194889**

(71) Applicant: **Medtronic MiniMed, Inc.
Northridge, CA 91325 (US)**

(72) Inventors:
• **Castaneda, Javier
  Northridge, 91325 (US)**
• **Cohen, Ohad
  Northridge, 91325 (US)**

(74) Representative: **Zimmermann & Partner
Patentanwälte mbB
P.O. Box 330 920
80069 München (DE)**

(54) **MODELING TARGETS OF CONTINUOUS GLUCOSE MONITORING METRICS FOR GLYCEMIC CONTROL**

(57) Disclosed herein are techniques for blood glucose management. In one example, a processor-implemented method includes receiving an input of a target value of a first continuous glucose monitoring (CGM) metric, estimating a target value of at least a second CGM metric that corresponds to the target value of the first CGM metric, and providing the estimated target value of at least the second CGM metric to a user. In some examples, the processor-implemented method also includes determining that the estimated target value of at least the second CGM metric meets a predetermined criterion, and configuring an insulin delivery system based on the target value of the first CGM metric.

*FIG. 1*

**Description**

CROSS-REFERENCE TO RELATED APPLICATION

This application claims the benefit of and priority to U.S. Patent Application Serial No. 19/194,889, filed April 30, 2025 and titled "MODELING TARGETS OF CONTINUOUS GLUCOSE MONITORING METRICS FOR GLYCEMIC CONTROL," which claims the benefit of and priority to U.S. Provisional Patent Application No. 63/648,352, filed May 16, 2024 and titled "MODELING TARGETS OF CONTINUOUS GLUCOSE MONITORING METRICS FOR GLYCEMIC CONTROL," each of which are hereby incorporated by reference in their entirety for all purposes.

TECHNICAL FIELD

The present disclosure relates generally to blood glucose management.

BACKGROUND

The pancreas of a healthy person can produce and release insulin into the blood stream in response to elevated blood glucose levels. More specifically, beta cells ($\beta$-cells) in the pancreas can produce and secrete insulin into the blood stream as needed. If $\beta$-cells become incapacitated or die, a condition known as Type 1 diabetes mellitus, insulin may need to be provided to the diabetic patient's body using, for example, a syringe, a pen, a pump, or another infusion delivery device of a blood glucose level management system, to maintain health or life.

Some blood glucose level management systems may be closed-loop systems that may include a pump automatically or semi-automatically controlled to deliver insulin to the patient. Some blood glucose level management systems may require manual administration of insulin based on dosage determined by an insulin calculator. The delivery of insulin to the patient can be controlled to occur at times and in amounts that are determined based on, for example, the amount of carbohydrates taken by a patient and/or real-time measurements of glucose levels by a glucose sensor, such as a continuous glucose monitor (CGM). Some blood glucose level management systems may deliver glucose and/or glucagon, in addition to the delivery of insulin, for controlling the blood glucose levels of the patient (e.g., in a hypoglycemic context).

SUMMARY

This disclosure relates generally to blood glucose management. More specifically, techniques disclosed herein relate to systems and methods for determining appropriate targets for continuous glucose monitoring (CGM) metrics to guide automated insulin delivery (AID) systems for glycemic control. Techniques disclosed herein may be practiced in a variety of ways, such as using a server, a user device, a processor-implemented method, a system comprising one or more processors and one or more processor-readable media, and/or one or more (non-transitory) processor-readable media.

According to certain embodiments, a method may include receiving an input of a target value of a first continuous glucose monitoring (CGM) metric, estimating a target value of at least a second CGM metric that corresponds to the target value of the first CGM metric, and providing the estimated target value of at least the second CGM metric to a user.

According to certain embodiments, a method may include classifying, using a classifier, continuous glucose monitoring (CGM) data samples meeting and not meeting a target value of a first CGM metric as samples meeting or not meeting each threshold target of a plurality of threshold targets of a second CGM metric; determining, for each threshold target of the plurality of threshold targets of the second CGM metric, a true positive rate (TPR) and a false positive rate (FPR) of the classifier; and identifying a target value of at least the second CGM metric based on the TPR and the FPR of the classifier for each threshold target of the plurality of threshold targets of the second CGM metric.

This summary is neither intended to identify key or essential features of the claimed subject matter, nor is it intended to be used in isolation to determine the scope of the claimed subject matter. The subject matter should be understood by reference to appropriate portions of the entire specification of this disclosure, any or all drawings, and each claim. The foregoing, together with other features and examples, will be described in more detail below in the following specification, claims, and accompanying drawings.

BRIEF DESCRIPTION OF THE DRAWINGS

The above and other aspects and features of the disclosure will become more apparent in view of the following detailed description when taken in conjunction with the accompanying drawings wherein like reference numerals identify like elements.

FIG. 1 illustrates an example of a blood glucose level management system according to certain embodiments.

FIG. 2 is a block diagram of an example of a blood glucose level management system according to certain embodiments.

FIG. 3 illustrates an example of a continuous glucose monitoring (CGM) system according to certain embodiments.

FIGS. 4A and 4B depict an example of an integrated glucose sensor device according to certain embodiments.

FIG. 5 depicts an example of an insulin delivery device according to some embodiments.

FIGS. 6A-6C illustrates examples of results of continuous glucose monitoring.

FIG. 7 illustrates an example of the recommended targets of a time-in-range metric for evaluating a glycemic control system.

FIG. 8 shows a summary of the above-described example of CGM dataset used to determine the target of a CGM metric based on the target of another CGM metric according to certain embodiments.

FIG. 9 shows another summary of the example of CGM dataset used to determine the target of a CGM metric based on the target of another CGM metric according to certain embodiments.

FIG. 10 includes a diagram illustrating an example of a receiver operating characteristics (ROC) curve.

FIG. 11 includes a table illustrating examples of the determined target values of certain time-in-range metrics that may correspond to different GMI target values according to certain embodiments.

FIGS. 12A and 12B show results of examples of Pearson correlation analysis of six time-in-range metrics and the GMI metric for users 15 years old or younger and users older than 15, respectively.

FIGS. 12C and 12D show results of principal component analysis (PCA) analysis of six time-in-range metrics and the GMI metric for users 15 years old or younger and users older than 15, respectively.

FIG. 13 includes a table showing examples of the results of estimating targets of TB54 associated with various TB70 targets according to certain embodiments.

FIG. 14 includes a flowchart illustrating an example of a method of determining a target value of a CGM metric that corresponds to a target value of another CGM target according to certain embodiments.

FIG. 15 includes a flowchart illustrating an example of a processor-implemented method for providing a target value of a CGM metric that corresponds to a target value of another CGM target to a user according to certain embodiments.

FIG. 16 is a block diagram of an example of a computer system that can be utilized to implement some embodiments described herein.

The figures depict embodiments of the present disclosure for purposes of illustration only. One skilled in the art will readily recognize from the following description that alternative embodiments of the structures and methods illustrated may be employed without departing from the principles, or benefits touted, of this disclosure.

In the appended figures, similar components and/or features may have the same reference label. Further, various components of the same type may be distinguished by following the reference label by a dash and a second label that distinguishes among the similar components. If only the first reference label is used in the specification, the description is applicable to any one of the similar components having the same first reference label irrespective of the second reference label.

DETAILED DESCRIPTION

Techniques disclosed herein relate generally to blood glucose management. More specifically, techniques disclosed herein relate to systems and methods for determining appropriate targets for continuous glucose monitoring (CGM) metrics to guide automated insulin delivery (AID) systems for glycemic control.

Diabetes mellitus is a disease of the glucose regulatory system of a patient, where the naturally produced insulin in the body may not be sufficient to control the glucose level in the blood stream of the patient, due to insufficient production of insulin and/or insulin resistance. Therefore, a diabetic patient may need to receive insulin from a pump or another delivery device, such as an injection or infusion device (e.g., a syringe), to control the glucose level in the patient's blood stream. To control the glucose level, a diabetic patient's therapy routine may generally include dosages of basal insulin and bolus insulin. Basal insulin, also referred to as background insulin, may include continuous or constant release of small amounts of insulin to keep blood glucose levels at consistent levels during long time periods. Bolus insulin may be taken specifically before, at, or after mealtimes or other times where there may be a rapid increase in the blood glucose level.

The dose of insulin to be delivered may be determined based on, for example, the carbohydrate count of a meal and/or the glucose levels of the patient measured using a glucose monitor, such as a fingerstick blood glucose measurement device or a continuous glucose monitoring (CGM) sensor. In one example, to counteract an increase in a patient's blood glucose level resultant from the consumption of a meal (or drink), insulin of a certain dose (referred to as a meal bolus) may be delivered to the patient prior to, contemporaneously with, or shortly after the start of the meal. The dose of the insulin may be determined using an insulin calculator (e.g., an App on a user device) that may consider factors such as the intake of carbohydrates, insulin sensitivity factor (ISF) of the patient, the patient's physiological condition (including the current glucose level), the target glucose range, and the like, and may indicate the number of units of insulin to be delivered. Too much insulin can lead to hypoglycemia, while too little insulin can lead to hyperglycemia.

There have been some recommended targets for glycemic control for Type 1 diabetes patients, such as a glucose management indicator (GMI) less than about 7% (or about 53 mmol/mol), over 70% of time within the range (TIR) of 70-180 mg/dL, less than 25% of time above 180 mg/dL (TA180), less than 5% of time above 250 mg/dL (TA250), less than 4% of time below 70 mg/dL (TB70), and less than 1% of time below 54 mg/dL (TB54). There is also a growing interest in a newly proposed metric, the percentage of time in a tight glucose range of 70-140 mg/dL (TITR), as a potential indicator of optimal CGM euglycemia. But currently there is no universally agreed-upon international target for TITR in the management of Type 1 diabetes.

Automated insulin delivery (AID) systems, such as Medtronic MiniMedTM 780G, have shown good performance in glycemic control, including the feasibility of achieving GMI targets close to 6.5% or lower. However, in many glycemic control systems, there may need to be some trade-offs in achieving or exceeding these recommended targets as efforts to improve one glycemic metric may have mixed effects on other CGM glycemic metrics. For example, increasing insulin doses may lower the GMI, TA180, and TA250, but may potentially increase the TB70 and TB54. Therefore, it may be desirable to determine whether meeting a specific glycemic target (e.g., a certain GMI target) may correspond to the compliance with certain targets of other glycemic metrics, such as the TIR, TITR, TB70, TB54, TA180, or TA250 targets, such that appropriate targets may be expected by the patients and/or may be used to control the glycemic control systems (e.g., an AID system) to achieve the desired results for specific patients. In addition, it may be desirable to explore the dimensionality of these CGM metrics to establish implications of, for example, targets of the time in various ranges based on GMI targets.

According to certain embodiments, techniques for determining the corresponding targets of one or more CGM metrics based on the target of another CGM metric are disclosed. In one example, a classifier may be trained to classify CGM data samples, and the classification results of the classifier may be used to determine whether a performance of a glycemic control system meeting a certain target of a first glycemic metric (e.g., GMI) can correspond to a performance meeting a threshold target of a second glycemic metric (e.g., TIR). For example, for a given target of the first glycemic metric (e.g., GMI less than 7%, less than 6.8%, less than 6.6%, or less than 6.5%), the classification results for different threshold target levels of the second glycemic metric (e.g., TIR, TITR, TB70, TB54, TA180, or TA250) may be determined and used to generate a receiver operating characteristic (ROC) curve. Based on the ROC curve, an appropriate threshold target level of the second glycemic metric that may maximize both the sensitivity and specificity of the classification (e.g., the threshold target level associated with a point on the ROC curve that is closest to the top-left corner) may be selected. The selected threshold target level of the second glycemic metric that may maximize both the sensitivity and specificity of the classification may be used as the target of the second glycemic metric that may be associated with or correspond to the target of the first glycemic metric. In this way, corresponding target values and possible trade-offs between two or more different CGM metrics may be determined and used for setting patient expectations and setting the control targets of glycemic control systems. The target of the second glycemic metric can be for a population or may be personalized.

In addition, dimension or parameter reduction techniques (e.g., principal component analysis (PCA)) and correlation techniques (e.g., pairwise Pearson correlation) may be used to determine the association between the GMI and the various time-in-range metrics based on the CGM data samples, and reduce the dimensionality of these CGM metrics while preserving relevant information (e.g., majority of the variance) inherent in the CGM data samples. For example, the time-in-range metrics and the GMI metric may be clustered to identify metrics that are associated with GMI, such as belonging to the same dimension or the orthogonal dimension with respect to GMI, thereby validating the determination of targets for time in various ranges associated with the GMI targets.

Techniques disclosed herein may be implemented on a server, a computer, a user device (e.g., a smartphone), a medical device (e.g., a CGM sensor or an insulin delivery device), and the like. In one example, a user app implementing techniques disclosed herein may be executed on the user device to provide a user with the corresponding targets of different CGM metrics based on a target of a CGM metric entered or selected by the user, so that the user may understand the expected glycemic control results and may select an appropriate target that may be suitable for the user to configure an AID system.

In the following description, for the purposes of explanation, specific details are set forth in order to provide a thorough understanding of examples of the disclosure. However, it will be apparent that various examples may be practiced without these specific details. For example, devices, systems, structures, assemblies, methods, and other components may be shown as components in block diagram form in order not to obscure the examples in unnecessary detail. In other instances, well-known devices, processes, systems, structures, operations, and techniques may be shown without necessary detail or may not be shown, in order to avoid obscuring the examples. The figures and description are not intended to be restrictive. The terms and expressions that have been employed in this disclosure are used as terms of description and not of limitation, and there is no intention in the use of such terms and expressions of excluding any equivalents of the features shown and described or portions thereof. The word "example" is used herein to mean "serving as an example, instance, or illustration." Any embodiment or design described herein as "example" is not necessarily to be construed as preferred or advantageous over other embodiments or designs.

Carbohydrates in food intake may be broken down into glucose (sugar) in the stomach and/or intestine, and may be absorbed at the small intestine and/or large intestine into the blood stream. The blood stream may transport glucose to the capillaries of the body, where some glucose may diffuse into the interstitial fluid between cells (e.g., fat or muscle cells), which may use glucose for energy. The endocrine system of the human body that directs and regulates the functions and activities of the body (working with the nervous system) may secrete chemicals that transmit messages to tissues and organs. For example, endocrine glands or organs may release hormones into the blood stream for transportation to target cells with receptors. In one example, insulin may be made by the β-cells of pancreas, and may, for example, increase the glucose uptake, enhance glucose utilization, stop hepatic glucose production, stimulate glycogen formation in liver and skeletal muscle, promote protein synthesis, and increase fat storage.

Insulin may function as a key that can unlock cells and help glucose to move into cells where the glucose may be used for energy. Without insulin, glucose may not be able to enter cells to be used for energy, and may build up in the interstitial fluid and blood stream. A healthy pancreas may continuously release a small amount of regular human insulin 24 hours a day, including between meals and during sleep. The small amount of insulin may match the liver's release of glucose. A healthy pancreas may also secrete a larger amount of insulin after food intake to match the amount of food intake.

The liver of a person may absorb excessive glucose from digestion and convert excessive glucose to glycogen for storage so that glucose may be released back to the blood stream when needed. For example, when the blood glucose level is low, the α-cells of the pancreas may secrete glucagon. Glucagon may cause the liver to release the stored form of glucose (glycogen) into the blood stream to help increase the glucose level. The balance between insulin secreted by the β-cells and the glucagon secreted by the α-cells may help to maintain the normal blood glucose level, such as in the range of about 80-140 mg/dL before meals.

The naturally produced insulin in the body of a diabetic patient may not be sufficient to control the glucose level in the blood stream of the patient, due to insufficient production of insulin and/or insulin resistance. Therefore, a diabetic patient may need to receive insulin from a pump or another delivery device such as an injection or infusion device to control the glucose level in the patient's blood stream. To control the glucose level, a diabetic patient's therapy routine may generally include dosages of basal insulin and bolus insulin. Dosages of insulin to be delivered may be determined based on, for example, the carbohydrate count of a meal, and/or the glucose level of the patient measured using a glucose monitor, such as a continuous glucose monitor (CGM).

In some implementations (e.g., in a closed-loop system), the insulin delivery device may communicate with or otherwise use a sensor device (including but not limited to a CGM) to perform various measurements for a patient. In one example, the sensor device may include subcutaneous implanted electrodes to concurrently monitor the patient's response to meals and insulin introduced by the insulin delivery device. The sensor device and the insulin delivery device may be in a communication network (wired or wireless) with one or more processors and/or patient devices (such as a patient's smartphone equipped with an application or other software) to create an overall system for monitoring a patient disease state and for facilitating treatment thereof.

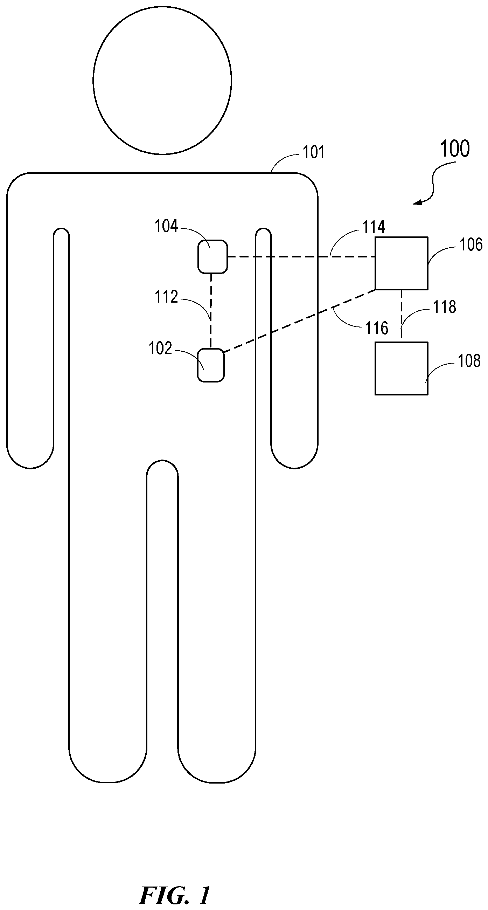

FIG. 1 illustrates an example of a blood glucose level management system 100 according to certain embodiments. Blood glucose level management system 100 may be used to monitor and regulate the blood glucose level of a patient 101. In the illustrated example, blood glucose level management system 100 may include a delivery device 102, a monitoring device 104, a computing device 106, and an optional remote/cloud computing system 108. Delivery device 102, monitoring device 104, and computing device 106 may be embodied in various ways, including being disposed in one or more device housings. For example, in some embodiments, all of devices 102-106 may be disposed in a single device housing. In some embodiments, each of devices 102-106 may be disposed in a separate device housing. In some embodiments, two or more of devices 102-106 may be disposed in the same device housing. In some embodiments, a single device 102, 104, or 106 may have two or more parts that are disposed in two or more housings. For example, monitoring device 104 may include an on-body part and a display and control part communicated with the on-body part through wires or wirelessly. Delivery device 102 may include an on-body site (e.g., including a cannula) and a part that includes a reservoir, a pump, and a control unit. These and other embodiments, and combinations thereof, are contemplated to be within the scope of the present disclosure.

Blood glucose level management system 100 may include a plurality of communication links, such as communication links 112-118. Communications links 112-118 may each be a wired connection and/or a wireless connection. In embodiments where two devices are located in a same housing, the communication link may include, for example, wires, cables, and/or communication buses on a printed circuit board, among other things. In embodiments where two devices are separate from each other in different device housings, the communication links may be wired and/or wireless connections. Wired connections may include, for example, an Ethernet connection, a Universal Serial Bus (USB) connection, and/or another type of physical connection. Wireless connections may include, for example, a cellular connection, a Wi-Fi connection, a Bluetooth® connection, a mesh network connection, and/or another type of connection using a wireless communication protocol. Some embodiments of communication links 112-118 may use direct connections, such as Bluetooth® connections, and/or may use connections that route through one or more networks or network devices (not shown), such as an Ethernet network, a Wi-Fi network, a cellular network, a satellite network, an intranet, an extranet, the Internet, and/or the Internet backbone, among other types of networks. Various combinations of wired and/or wireless connections may be used for communication links 112-118.

Delivery device 102 may be configured to deliver a therapeutic substance to patient 101. The therapeutic substance may include, for example, insulin, HIV drugs, drugs to treat pulmonary hypertension, iron chelation drugs, pain medications, anti-cancer treatments, medications, vitamins, hormones, a nutritional supplement, a dye, a tracing medium, a saline medium, a hydration medium, and the like. Delivery device 102 may be secured to patient 101 (e.g., to the body or clothing of patient 101) or may be at least partially implanted in the body of patient 101. In some embodiments, the delivery device 102 may include a reservoir, an actuator, a delivery mechanism, and a cannula (not shown). The reservoir may be configured to store an amount of the therapeutic substance. In some embodiments, the reservoir may be refillable or replaceable. The actuator may be configured to drive the delivery mechanism. In some examples, the actuator may include a motor, such as an electric motor. The delivery mechanism may be configured to move the therapeutic substance from the reservoir through the cannula. In some examples, the delivery mechanism may include a pump and/or a plunger. The cannula may facilitate a fluidic connection between the reservoir and the body of patient 101. The cannula and/or a needle may facilitate delivery of the therapeutic substance to a tissue layer, vein, interstitial fluid, or body cavity of patient 101. During operation, the actuator, in response to a signal (e.g., a command signal), may drive the delivery mechanism, thereby causing the therapeutic substance to move from the reservoir, through the cannula, and into the body of patient 101.

The components of delivery device 102 described above are merely provided as an example. Delivery device 102 may include other components, such as, without limitation, a power supply, a communication transceiver, one or more processors or other computing resources, memory devices, and/or user interfaces (e.g., buttons, keys, display, etc.), among other things. In some implementations, delivery device 102 may host an App (e.g., an insulin calculator) that may calculate the desired amount of therapeutic substance to be delivered to patient 101. Persons skilled in the art will recognize various implementations of delivery device 102 and the components of such implementations. All such implementations and components are contemplated to be within the scope of the present disclosure.

Monitoring device 104 may be configured to detect a physiological condition (e.g., a glucose concentration level) of patient 101 and may also be configured to detect other physiological conditions. Monitoring device 104 may be secured to the body of patient 101 (e.g., to the skin of patient 101 via an adhesive) and/or may be at least partially implanted into the body of patient 101. Depending on the particular location or configuration, monitoring device 104 may be in contact with biological matter (e.g., interstitial fluid and/or blood) of patient 101.

Monitoring device 104 may include one or more sensors (not shown), such as, without limitation, electrochemical sensors, electrical sensors, and/or optical sensors. As persons skilled in the art will understand, an electrochemical sensor may be configured to respond to the interaction or binding of a biological marker to electrodes by generating an electrical signal based on, for example, a potential, conductance, current, and/or impedance of an electrical path through the electrodes. The electrodes may include a material selected to interact with a particular biomarker, such as glucose. The potential, current, conductance, and/or impedance may correlate with a concentration of the particular biomarker. In one example, the electrochemical sensor may include a glucose limiting membrane (GLM) that limits the amount of glucose and oxygen delivered to a glucose oxidase (GOx) layer of a working electrode of the sensor to ensure that the reactions are glucose limited. The GOx layer or another active enzyme layer on the working electrode of the sensor may break down glucose and oxygen into gluconic acid and hydrogen peroxide. The generated peroxide molecules may interact with the working electrode to break down hydrogen peroxide into two hydrogen ions, oxygen, and two electrons at the surface of the working electrode, when a voltage signal is supplied to the working electrode. The electrical charges may be forced to move between electrodes (e.g., between the working electrode and counter electrode), thereby generating a sensor current signal (Isig) that can be measured by sensor electronics. Other signals such as the counter voltage (Vcntr, the voltage potential difference between the counter electrode and the working electrode), electrochemical impedance spectroscopy (EIS) at different frequencies, and the like, may also be measured. The signals measured using the sensor, including the Isig, Vcntr, and EIS, may be processed (e.g., filtered or transformed) to generate some other signals or parameters, such as filtered Isig signals, real and imaginary impedance at various frequencies, and the like. These signals and/or the processed parameters may be used in one or more sensor glucose (SG) models (e.g., machine learning models or mathematical models) to determine SG values that may be estimations of the blood glucose (BG) levels of the patient.

As persons skilled in the art would understand, an electrical sensor may be configured to respond to an electrical biosignal by generating an electrical signal based on an amplitude, frequency, and/or phase of the electrical biosignal. The electrical biosignal may include a change in electric current produced by the sum of an electrical potential difference across a tissue, such as the nervous system, of patient 101. In some embodiments, the electrical biosignal may include portions of a potential change produced by the heart of patient 101 over time (e.g., recorded as an electrocardiogram) that may be indicative of a glucose level of patient 101. An optical sensor may be configured to, for example, respond to the interaction or binding of a biological marker to a substrate by generating an electrical signal based on change in luminance of the substrate. In one example, the substrate may include a material selected to fluoresce in response to contact with a selected biomarker, such as glucose. The fluorescence may be proportional to a concentration of the selected biomarker.

In some embodiments, monitoring device 104 may include other types of sensors that may be worn, carried, or coupled to patient 101 to measure activity of patient 101 that may influence the glucose levels or glycemic response of patient 101. As an example, the sensors may include an acceleration sensor configured to detect an acceleration of patient 101 or a portion of the patient 101, such as the person's hands or feet, the position changes of which may be associated with an activity of patient 101. For example, the acceleration or movement (or lack thereof) of the body portion of patient 101 may be indicative of exercise, sleep, or food/beverage consumption activity of patient 101, which may influence the glycemic response of patient 101. In some embodiments, the sensors may measure heart rate and/or body temperature, which may indicate an amount of physical exertion experienced by patient 101. In some embodiments, the sensors may include a Global Positioning System (GPS) receiver which may detect GPS signals to determine a location of patient 101.

The sensors described above are merely provided as examples. Other sensors or types of sensors for monitoring physiological condition, activity, and/or location, among other things, will be recognized by persons skilled in the art and are contemplated to be within the scope of the present disclosure. For any sensor, the signal provided by a sensor may be referred to herein as a "sensor signal." As used herein, the term "sensed data" may mean and include the information represented by a sensor signal or by a pre-processed sensor signal. In some embodiments, sensed data may include glucose levels of patient 101, acceleration of a part of patient 101, heart rate of patient 101, temperature of patient 101, and/or geolocation (e.g., GPS location) of patient 101, among other things. Monitoring device 104 may communicate sensed data to delivery device 102 via communication link 112 and/or to computing device 106 via communication link 114. Use of sensed data by delivery device 102 and/or by computing device 106 is described in more detail below.

In some embodiments, monitoring device 104 may include components and/or circuitry configured to pre-process sensor signals. Pre-processing may include, for example, amplification, filtering, attenuation, scaling, isolation, normalization, transformation, sampling, and/or analog-to-digital conversion, among other things. In some embodiments, monitoring device 104 may host an App for processing the sensor signals. In some embodiments, monitoring device 104 may include a wired or wireless transceiver as described above for transmitting the sensor signals or receiving commands or instructions. Persons skilled in the art will recognize various implementations for such pre-processing, including, without limitation, implementations using processors, controllers, integrated circuits, application specific integrated circuits (ASICs), hardware, firmware, programmable logic devices, and/or machine-executable instructions, among others. The types of pre-processing and their implementations are merely provided as examples. Other types of pre-processing and implementations are contemplated to be within the scope of the present disclosure. In some embodiments, monitoring device 104 may not perform pre-processing.

Computing device 106 may provide processing capabilities and may be implemented in various ways. In some embodiments, computing device 106 may be a consumer device, such as a smartphone, a computerized wearable device (e.g., a smartwatch), a tablet computer, a laptop computer, or a desktop computer, among others, or may be a special purpose device (e.g., a portable control device) provided by, for example, the manufacturer of delivery device 102. In some embodiments, computing device 106 may be processing circuitry that may be integrated with another device, such as delivery device 102. In some embodiments, computing device 106 may be secured to patient 101 (e.g., to the body or clothing of patient 101), may be at least partially implanted into the body of patient 101, and/or may be held by patient 101.

For each of the embodiments of computing device 106, computing device 106 may include various types of logic circuitry, including, but not limited to, microprocessors, controllers, digital signal processors (DSPs), application specific integrated circuits (ASICs), field-programmable gate arrays (FPGAs), central processing units (CPU), graphics processing units (GPU), programmable logic devices, memory (e.g., random access memory, volatile memory, non-volatile memory, etc.), or other discrete or integrated logic circuitry, as well as combinations of such components. The term "processing circuitry" may generally refer to any of the foregoing logic circuitry, alone or in combination with other logic circuitry, or any other circuitry for performing computations.

Some aspects of delivery device 102, monitoring device 104, and computing device 106 have been described above. One or more of devices 102-106 may include a user interface (not shown) that presents information to patient 101 and/or receives information from patient 101. The user interface may include a graphical user interface (GUI), a display device, a keyboard, a touchscreen, a speaker, a microphone, a vibration motor, buttons, switches, and/or other types of user interfaces. Persons skilled in the art will recognize various types of user interfaces that may be used, and all such user interfaces are contemplated to be within the scope of the present disclosure. For example, where computing device 106 is a consumer device such as a smart phone, tablet computer, laptop computer, or the like, the user interfaces would include a display device, a physical and/or virtual keyboard, and/or audio speakers provided by such consumer devices, among other things. In some embodiments, a user interface may notify patient 101 of sensed data (e.g., glucose level) and/or insulin delivery data (e.g., rates of historic, current, or future insulin delivery) and may present alerts to patient 101. In some embodiments, a user interface may receive inputs from patient 101, which may include, for example, a requested change in insulin delivery setting and/or a meal indication, among other things. The descriptions and embodiments above regarding user interfaces are merely provided as examples, and other types and other uses of user interfaces are contemplated to be within the scope of the present disclosure.

In one specific example, the communications between devices 102-106 and cooperation between devices 102-106 may be used for insulin delivery. As depicted in FIG. 1 and as described above, devices 102-106 may communicate with each other via communication links 112-116. In some embodiments, computing device 106 may control operations of delivery device 102 and/or monitoring device 104. For example, computing device 106 may generate one or more signals (e.g., a command signal) that cause delivery device 102 to deliver insulin to patient 101, for example, as a basal dosage and/or a bolus dosage. In some embodiments, computing device 106 may receive data associated with insulin delivery (e.g., insulin delivery data) from delivery device 102 and/or receive sensed data (e.g., glucose levels) from monitoring device 104, and may perform computations based on the insulin delivery data, the sensed data, and/or other data to control delivery device 102. Insulin delivery data may include, but is not limited to, the type of insulin being delivered, historical insulin delivery rates and/or amounts, current insulin delivery rate and/or amount, insulin delivery time, and/or user inputs affecting insulin delivery. As persons skilled in the art will understand, in a closed-loop operating mode, computing device 106 may communicate dosage commands to delivery device 102 based on, for example, a difference between a current glucose level in the body of patient 101 (e.g., received from monitoring device 104) and a target glucose level (e.g., determined by computing device 106 or set on delivery device 102). The dosage commands may indicate an amount of insulin to be delivered and/or a rate (or time) of insulin delivery, and may regulate the current glucose level toward the target glucose level.

Remote/cloud computing system 108 may be any proprietary remote/cloud computing system or a commercial cloud computing system including one or more server computing devices. Remote/cloud computing system 108 may provide alternative or additional computing resources as needed when the computing resources of a client computing device (e.g., computing device 106) are not sufficient. Computing device 106 and remote/cloud computing system 108 may communicate with each other through a communication link 118, which may traverse one or more communication networks (not shown). The communication networks may include, for example, an Ethernet network, a Wi-Fi network, a cellular network, a satellite network, an intranet, an extranet, the Internet, and/or the Internet backbone, among other types of networks. Persons skilled in the art will recognize implementations for remote/cloud computing system 108 and how to interface with such systems through various types of networks. For example, remote/cloud computing system 108 may include an array of processing circuitry and may execute machine-readable instructions. Such implementations, interfaces, and networks are contemplated to be within the scope of the present disclosure.

In some embodiments, remote/cloud computing system 108 may make a therapy determination (e.g., an insulin amount or adjusted insulin amount), and may communicate the therapy to delivery device 102 via computing device 106. In some embodiments, computing device 106 may make the therapy determination and communicate it to delivery device 102. In some embodiments, monitoring device 104 may make the therapy determination and communicate it to delivery device 102 either directly or through an intermediary such as computing device 106.

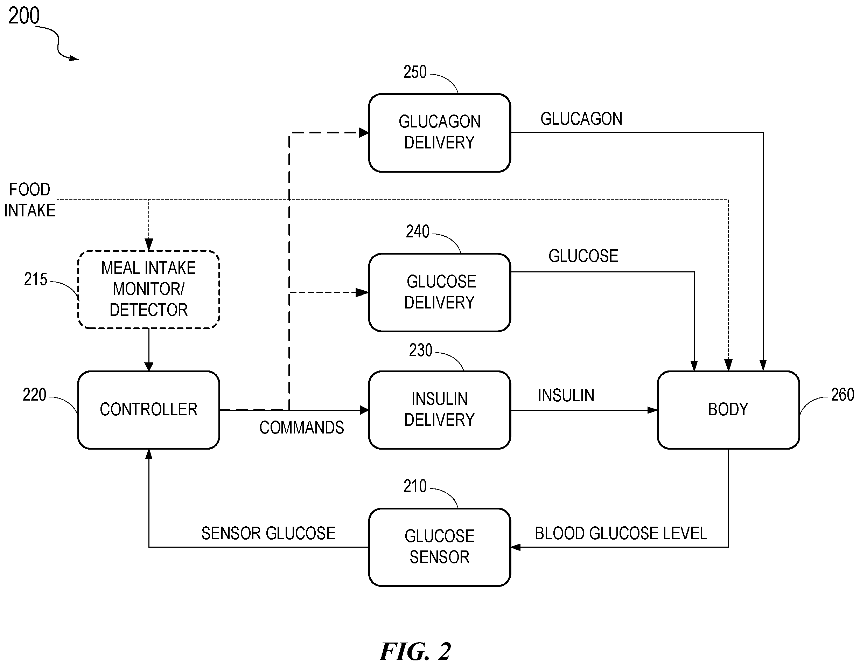

FIG. 2 is a block diagram of an example of a blood glucose level management system 200 according to certain embodiments. In the illustrated example, blood glucose level management system 200 may include a glucose sensor subsystem 210, a controller 220, an insulin delivery subsystem 230, a glucose delivery subsystem 240, and a glucagon delivery subsystem 250. Glucose sensor subsystem 210 may generate sensor glucose (SG) signals (e.g., SG levels) that may be the estimations of blood glucose levels in a body 260, and may provide the SG signals to controller 220. Controller 220 may receive the SG signals and generate commands to insulin delivery subsystem 230, and, in some implementations, glucose delivery subsystem 240 and/or glucagon delivery subsystem 250. Insulin delivery subsystem 230 may receive commands from controller 220 and deliver insulin to body 260 according to the commands. In some embodiments, glucose delivery subsystem 240 may receive commands from controller 220 and provide glucose into body 260 according to the commands. In some embodiments, glucagon delivery subsystem 250 may receive commands from controller 220 and deliver glucagon into body 260 according to the commands.

In some implementations, glucose sensor subsystem 210 may include a glucose sensor, sensor electronics configured to generate SG signals, a sensor communication system configured to send the SG signals to controller 220, and a housing for the sensor electronics and the sensor communication system. The glucose sensor may measure blood glucose levels, for example, directly from a blood stream, or indirectly via interstitial fluid using a subcutaneous sensor as described in more detail below.

Controller 220 may include electrical components and software to generate commands for insulin delivery subsystem 230, glucose delivery subsystem 240, and/or glucagon delivery subsystem 250. Controller 220 may include a controller communication system to receive the sensor signal and provide the commands to insulin delivery subsystem 230, glucose delivery subsystem 240, and/or glucagon delivery subsystem 250. In some implementations, controller 220 may implement a glucose calculator. In some implementations, controller 220 may include a user interface and/or operator interface (not shown) comprising a data input device and/or a data output device. Such a data output device may, for example, generate signals to initiate an alarm and/or include a display or printer for showing status of controller 220 and/or a patient's vital indicators. Such a data input device may include dials, buttons, pointing devices, manual switches, alphanumeric keys, a touch-sensitive display, combinations thereof, and/or the like for receiving user and/or operator inputs. Such a data input device may be used for scheduling and/or initiating insulin bolus injections for meals, for example. It should be understood, however, that these are merely examples of input and output devices that may be a part of an operator and/or user interface and that claimed subject matter is not limited in these respects.

Insulin delivery subsystem 230 may include, for example, an infusion device and/or an infusion tube to infuse insulin into body 260. Similarly, glucose delivery subsystem 240 may include, for example, an infusion device and/or an infusion tube to infuse glucose into body 260. Likewise, glucagon delivery subsystem 250 may include, for example, an infusion device and/or an infusion tube to infuse glucagon into body 260. In some embodiments, the insulin, glucagon, and/or glucose may be infused into body 260 using a shared delivery system and/or infusion tube. In some embodiments, the insulin, glucagon, and/or glucose may be infused using an intravenous system for providing fluids to a patient (e.g., in a hospital or other medical environment). It should be understood, however, that certain example embodiments may include an insulin delivery subsystem 230 without a glucagon delivery subsystem 250 and/or without a glucose delivery subsystem 240. In some embodiments, each of insulin delivery subsystem 230, glucose delivery subsystem 240, and glucagon delivery subsystem 250 may include infusion electrical components to activate an infusion motor according to the commands from controller 220, an infusion communication system to receive commands from controller 220, and a delivery subsystem housing.

In some embodiments, controller 220 may be housed in a delivery subsystem housing, and an infusion communication system may comprise an electrical trace or a wire that carries the commands from controller 220 to the delivery subsystem. In some embodiments, controller 220 may be housed in a sensor system housing, and a sensor communication system may comprise an electrical trace or a wire that carries the sensor signal from sensor electrical components to controller electrical components. In some embodiments, controller 220 may have its own housing or may be included in a supplemental device. In some embodiments, controller 220 may be co-located with a delivery subsystem and a sensor system within a single housing. In some embodiments, a sensor, a controller, and/or infusion communication systems may utilize a cable; a wire; a fiber optic line; RF, IR, or ultrasonic transmitters and receivers; combinations thereof; and/or the like instead of electrical traces, just to name a few examples.

In some embodiments, blood glucose level management system 200 may also include a meal intake monitoring subsystem 215. Meal intake monitoring subsystem 215 may be used to log the amount of user food intake, or may automatically detect the amount of user food intake. For example, in some implementations, the user may enter the food items and/or the estimated amount of carbohydrates in a meal at the meal time. In some implementations, meal intake monitoring subsystem 215 may include sensors (e.g., cameras or accelerators) that may automatically detect a meal event, the food items in a meal, and/or the estimated amount of carbohydrates in the meal. The estimated amount of carbohydrates in the meal may be sent to controller 220, which may determine an appropriate amount of meal bolus and generate a command for insulin delivery subsystem 230 to deliver the meal bolus. In some implementations, meal intake monitoring subsystem 215 may not be used in blood glucose level management system 200, and the dosage for the meal bolus may be determined based on the measured glucose level.

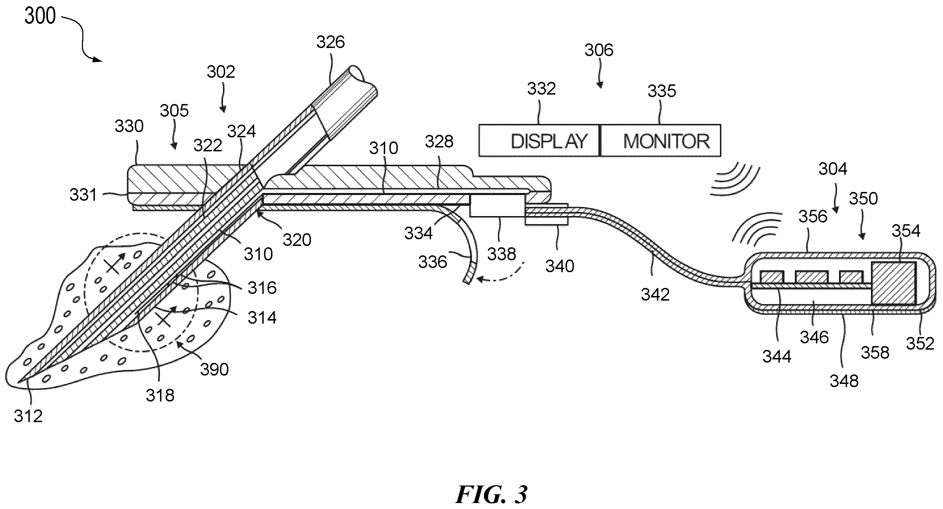

FIG. 3 is a perspective view of an example of a CGM system 300 according to certain embodiments. In the illustrated example, CGM system 300 may include a sensor set 302 provided for subcutaneous placement of an active portion of a flexible sensor 310, or the like, at a selected site in the body of a user. The subcutaneous or percutaneous portion of sensor set 302 includes a hollow, slotted insertion needle 326 having a sharpened tip 312, and a cannula 322. Sensor set 302 may facilitate accurate placement of flexible sensor 310 into the body of the user. Inside cannula 322 is a glucose sensing portion 318 of flexible sensor 310. Glucose sensing portion 318 includes one or more sensor electrodes 316 that may be exposed to the user's bodily fluids 390, for example, through a window 314 formed in the cannula 322. Sensor electrodes 316 may include, for example, a counter electrode, a reference electrode, and one or more working electrodes.

The proximal part of flexible sensor 310 may be mounted in a mounting base 305 adapted for placement onto the skin of the user. In some embodiments, mounting base 305 can be a pad having an underside surface coated with a suitable pressure sensitive adhesive layer 334, with a peel-off paper strip 336 normally provided to cover and protect the adhesive layer 334, until sensor set 302 is ready for use. Mounting base 305 may include an upper layer 330 and a lower layer 331, with a connection portion 328 of flexible sensor 310 being sandwiched between upper layer 330 and lower layer 331. Connection portion 328 may include a forward section joined to glucose sensing portion 318 of flexible sensor 310, which may be folded angularly to extend downwardly through a bore 320 formed in lower layer 331 of mounting base 305. Optionally, adhesive layer 334 (or another portion of the apparatus in contact with in vivo tissue) may include an anti-inflammatory agent to reduce an inflammatory response and/or anti-bacterial agent to reduce the chance of infection. Insertion needle 326 may be adapted for slide-fit reception through a needle port 324 formed in upper layer 330 of mounting base 305 and through lower bore 320 in the lower layer 331 of mounting base 305. In some embodiments, insertion needle 326 may be withdrawn after insertion to leave glucose sensing portion 318 (including sensor electrodes 316) and/or cannula 322 in place at the selected insertion site.

Flexible sensor 310 of sensor set 302 may be connected to a sensor electronics device 304, which may be referred to as a transmitter. For example, mounting base 305 may be designed so that glucose sensing portion 318 may be joined to a connection portion 328 that terminates in conductive contact pads, or the like. Connection portion 328 and the contact pads may be adapted for an electrical connection to sensor electronics device 304 for determining the glucose level of the user in response to signals from sensor electrodes 316. Connection portion 328 may be connected electrically to sensor electronics device 304 by a connector block 338. Sensor electronics device 304 may include, for example, a housing 350 that supports a printed circuit board 344, batteries 354, and an antenna 346. In some embodiments, housing 350 may include an upper case 356 and a lower case 352 that are sealed by, for example, ultrasonic welding, to form a waterproof (or resistant) seal to permit cleaning by immersion (or swabbing) with water, cleaners, alcohol or the like. In some embodiments, upper case 356 and lower case 352 may be formed from a medical grade plastic. In alternative embodiments, upper case 356 and lower case 352 may be connected together by other methods, such as snap fits, sealing rings, RTV (silicone sealant) and bonded together, or the like, or formed from other materials, such as metal, composites, ceramics, or the like. In other embodiments, the assembly may be potted in epoxy or other moldable materials that is compatible with the electronics and reasonably moisture resistant. In some embodiments, sensor electronics device 304 may be connected to flexible sensor 310 through connector block 338 and a connector 340, and may be mounted onto mounting base 305. In some embodiments, sensor electronics device 304 may be connected to flexible sensor 310 through connector block 338, connector 340, and a cable 342. In some embodiments, lower case 352 may have a bottom surface coated with a suitable pressure sensitive adhesive layer 358, with a peel-off paper strip 348 normally provided to cover and protect the adhesive layer 358, until sensor electronics device 304 is ready for use.

Batteries 354 may include chargeable or non-chargeable batteries. In some embodiments, batteries 354 may include silver oxide battery cells. In other examples, different battery chemistries may be utilized, such as lithium based chemistries, alkaline batteries, nickel metalhydride, or the like, and a different number of batteries may be used. Batteries 354 may provide power to sensor set 302 via sensor electronics device 304 through, for example, connector block 338, connector 340, and/or cable 342.

Sensor electronics device 304 may include a processor, a transceiver unit, signal processing/conditioning circuits, and memory devices on printed circuit board 344. The processor may be configured to receive current and/or voltage signals from sensor electrodes 316 of flexible sensor 310. Sensor electrodes 316 may generate sensor signals indicative of a concentration of analyte (e.g., glucose) being measured. In one example, the sensor signals may include a current measured at a working electrode that may include a glucose oxidase (GOx) enzyme for catalyzing a reaction with glucose. The sensor signals may also include voltage signals measured at a counter electrode of sensor electrodes 316, or between a working electrode and a counter electrode (or reference electrode). In some examples, other electrical parameters, such as capacitance, resistance, impedance, and the like, may also be measured using sensor electrodes 316 inserted into the body of the user.

An example of the impedance parameters measured using sensor electrodes 316 may include electrochemical impedance spectroscopy (EIS) values. EIS may provide additional information in the form of sensor impedance and impedance-related parameters at different frequencies. Moreover, for certain ranges of frequencies, impedance and/or impedance-related data may be substantially glucose independent. Such glucose independence may enable the use of a variety of EIS-based markers or indicators for not only producing a robust, highly reliable sensor glucose value (e.g., through fusion methodologies), but also assessing the condition, health, age, and efficiency of individual electrode(s) of the overall sensor substantially independent of the glucose-dependent current signal. In one example, the analysis of the glucose-independent impedance data may provide information about the efficiency of a glucose sensor with respect to how quickly it hydrates and is ready for data acquisition. In addition, glucose-independent impedance data (using, e.g., values for 1 kHz real impedance) may provide information on potential occlusion(s) that may exist on a sensor membrane surface, where the occlusion(s) may temporarily block passage of glucose into the sensor and thus cause the signal to dip.

Furthermore, glucose-independent impedance data may provide information on loss of sensor sensitivity during extended wear, for example, using values of phase angle and/or imaginary impedance at 1 kHz and higher frequencies.

The processor of sensor electronics device 304 may receive the sensor signals and calibrate the sensor signals using a calibration function that may be determined, for example, using reference values. In some embodiments, parameters (e.g., calibration factor or sensor sensitivity) of the calibration function may be determined (e.g., using calibration data) or pre-determined and stored in the memory for use by the processor. For example, in some embodiments, the processor may implement or otherwise execute a calibration application module that calculates or otherwise determines calibration parameters based on the measurement values and references values (e.g., blood glucose values measured using, for example, a fingerstick blood glucose meter). Based on the sensor signals and the determined calibration function, the processor may estimate the blood glucose level of the user. In some embodiments, the processor may store the glucose measurements in the memory. The sensor measurement results may be displayed on a display of sensor electronics device 304 or may be sent to a receiver 306 for display.

The memory of sensor electronics device 304 may be any type of memory device and may be configured to store raw sensor signals from flexible sensor 310 and/or glucose measurements (e.g., SG values) produced by the processor, calibration parameters used to determine glucose measurements from sensor signals, or other data used and/or produced by the processor. The memory may further store software and/or firmware that is executable by the processor. In some embodiments, the memory may further include configuration information for configuring the operation of sensor electronics device 304.

The signal processing/conditioning circuits of sensor electronics device 304 may include one or more amplifiers, filters, current to voltage converters, integrators, analog-to-digital converters, potentiostat, and the like. The signal processing/conditioning circuits may filter the signals, apply a constant reference voltage level, amplify current or voltage signals, sample and convert analog current or voltage signals into digital data values, and the like.

The transceiver unit of sensor electronics device 304 may include a wireless transceiver that may send the glucose measurements determined by the processor and stored in the memory to receiver 306 using a suitable wireless communication protocol, such as Bluetooth (e.g., Bluetooth low energy (BLE)), Wi-Fi, WiMax, and the like. For example, the transceiver unit may send radio signals indicative of the glucose level at regular time periods (e.g., every 5 minutes) to provide real-time sensor glucose (SG) values. The transceiver unit may also receive data, such as configuration data and calibration data (e.g., reference glucose values), from receiver 306. In some embodiments, the transceiver unit of sensor electronics device 304 may send data to receiver 306 through a cable or wire.

Receiver 306 may include a monitor 335 for monitoring physiological characteristics readings and/or generating alert signals, and a display 332 for displaying physiological characteristics readings and/or alert signals. For example, SG values/graphs may be displayed on display 332 of receiver 306 so that a user can monitor the blood glucose level and administer insulin using an insulin pump. In various embodiments, receiver 306 may be implemented in a desktop computer, a pager, a television including communications capabilities, a laptop computer, a server, a network computer, a personal digital assistant (PDA), a smart phone, an infusion pump including a display, a glucose sensor including a display, a combined infusion pump/glucose sensor, and the like.

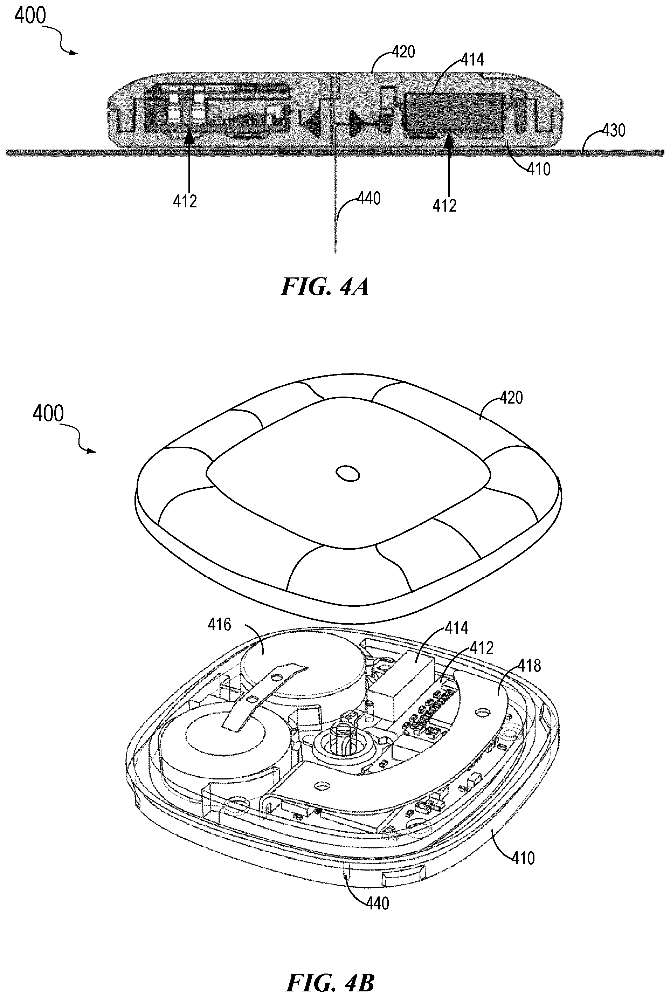

FIGS. 4A and 4B depict an example of an integrated glucose sensor device 400 according to certain embodiments, which may implement some of the examples disclosed herein. Glucose sensor device 400 may be an example of monitoring device 104, glucose sensor subsystem 210, or CGM system 300. In the illustrated example, glucose sensor device 400 may include a housing formed by a base 410 and a top cover 420, sensor electronics enclosed in the housing, an electrochemical sensor 440 partially within the housing and connected to the sensor electronics, and an adhesive patch 430 attached to base 410. Electrochemical sensor 440 may be an example of the electrochemical sensors described above, and may include multiple electrodes at a distal end that may be inserted into the subcutaneous layer of a user. A proximal end of electrochemical sensor 440 may be bent and held against a printed circuit board (PCB) 412 by a elastomer or rubber block 414, such that contacts on the proximal end of electrochemical sensor 440 may be physically and electrically connected to contact pads on PCB 412. The housing may be hermetically sealed to protect the sensor electronics from moisture. Electrochemical sensor 440 may be inserted into the subcutaneous layer of the user using an inserter. The inserter may include a needle and may enclose the entire glucose sensor device 400 before insertion. When activated (e.g., pushed), the inserter may move the entire glucose sensor device 400 toward the skin of the user to attach adhesive patch 430 to the skin of the user and insert the distal end of electrochemical sensor 440 into the subcutaneous layer of the user using the needle, where the needle may be retracted, for example, automatically after the distal end of electrochemical sensor 440 is inserted into the user.

The sensor electronics in the housing may include batteries 416, an antenna 418, and circuits on PCB 412. The circuits on PCB 412 may include, for example, a processor, a controller, a potentiostat, filters, amplifiers, a wireless (e.g., Bluetooth) transceiver, one or more memory devices, power conversion and management devices, or a combination thereof. Predicted in vivo characteristics of electrochemical sensor 440 and SG model(s) described above may be stored in the one or more memory devices. Before or after the distal end of electrochemical sensor 440 is inserted into the subcutaneous layer of the user, the sensor electronics may be activated to initialize the sensor for measuring sensor measurement data (e.g., Isig, Vcntr, EIS, etc.). The processor or controller may execute instruction code stored on the one or more memory device to obtain the sensor measurement data, process (e.g., filter, amplify, transform, etc.) the sensor measurement data, estimate SG values using the SG models and sensor measurement data, optionally blank certain estimated SG values, and transmit, via the wireless transceiver and antenna 418, the estimated SG values to a receiver (e.g., a smartphone or an insulin pump), as described above.

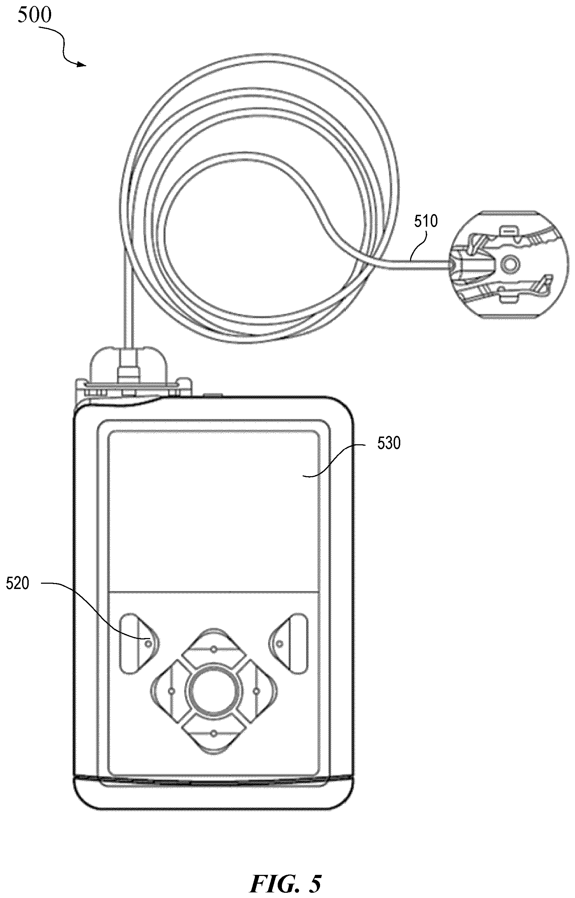

FIG. 5 depicts an example of a delivery device 500, which may implement a delivery device described above (e.g., delivery device 102 or insulin delivery subsystem 230). In an insulin delivery device implemented using delivery device 500, insulin delivery may be performed based on internal communication between a central computing module (e.g., a microprocessor of delivery device 500) and an insulin delivery module (e.g., including a microcontroller, a motor, and a pump). For instance, insulin delivery may be caused by the central computing module communicating a delivery command in the form of an electrical signal that travels via a communication fabric to the insulin delivery module. The central computing module may also be configured to communicate (e.g., via a transceiver) with a computing device (e.g., computing device 106 of FIG. 1) communicatively coupled to a remote or cloud computing system (e.g., remote/cloud computing system 108 of FIG. 1). Delivery device 500 may communicate various event data toward the remote or cloud computing system, which may communicate insulin delivery determinations toward delivery device 500, in accordance with the techniques described herein.

Delivery device 500 may provide insulin through a small tube 510 configured for fluidic connection with a cannula inserted subcutaneously. Delivery device 500 may be configured to deliver two types of dosages-a basal dosage, which can be delivered periodically (e.g., every five minutes) in tiny amounts throughout the day and night, and a bolus dosage to cover an increase in blood glucose from meals and/or to correct high blood glucose levels. In the depicted example, delivery device 500 includes a user interface having button elements 520 that can be manipulated to administer a bolus of insulin, to change therapy settings, to change user preferences, to select display features, and the like. Delivery device 500 may also include a display device 530 that can be used to present various types of information or data to the user. In accordance with aspects of the present disclosure, a user of delivery device 500 may use button elements 520 to input certain event data (e.g., event type, event start time, event details, etc.), and the user inputs can be confirmed using display device 530. Delivery device 500 of FIG. 5 is merely provided by way of example, and other types of insulin delivery devices and other techniques different from those described above are contemplated to be within the scope of the present disclosure.

Based on the glucose levels measured using CGMs (e.g., CGM system 300 or glucose sensor device 400), the performance of a glycemic control system, such as the human body or an AID system (e.g., including delivery device 500), may be determined using various metrics. For example, a glucose management indicator (GMI) may be used to describe the average (mean) glucose value measured using a CGM during a period of time (e.g., about 10 to 14 days). The GMI may be determined according to:

$$GMI\ (\text{percentage}) = 3.31 + 0.02392 \times (\text{glucose reading in mg/dL}).$$

A GMI below about 5.7% may indicate a normal condition, a GMI between about 5.7% and about 6.5% may indicate a prediabetes condition, whereas a GMI greater than about 6.5% may indicate a diabetes condition. The goal for most adults with Type 1 diabetes may be to keep the GMI less than about 7% (or about 154 mg/dL). Time-in-range metrics may be used to describe how often the glucose levels of a person are within different ranges.

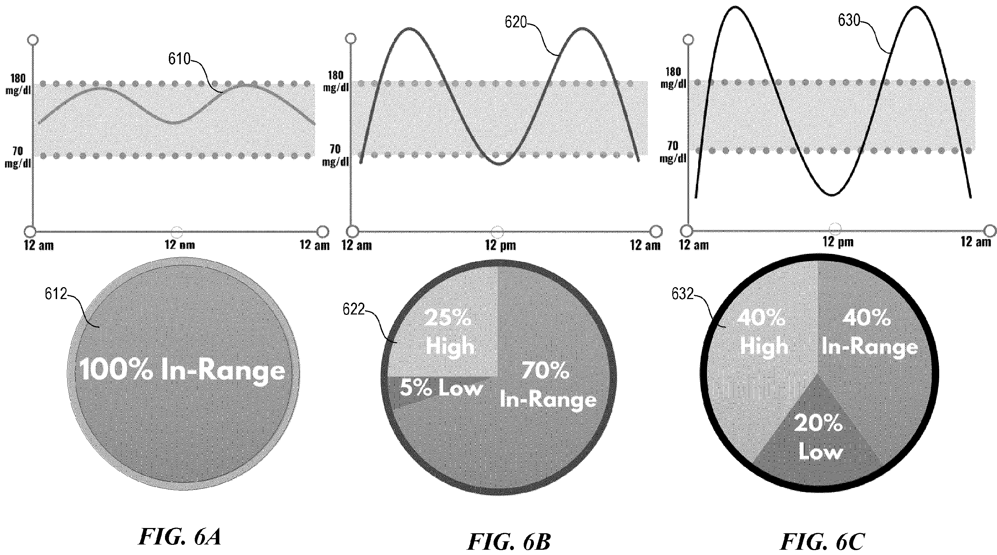

FIGS. 6A-6C illustrate examples of results of continuous glucose monitoring. In the example shown in FIG. 6A, a curve 610 shows the measured glucose levels within 24 hours, and a pie chart 612 shows the statistics of the measured glucose levels during the 24 hours. Curve 610 and pie chart 612 show that the measured glucose levels in the illustrated example are within the range of 70-180 mg/dL during 100% of the time in a day. In the example shown in FIG. 6B, a curve 620 shows the measured glucose levels within 24 hours, and a pie chart 622 shows the statistics of the measured glucose levels during the 24 hours. Curve 620 and pie chart 622 show that, within the 24 hours, the measured glucose levels in the illustrated example are within the range of 70-180 mg/dL during about 70% of the time, are below 70 mg/dL during about 5% of the time, and are above 180 mg/dL during about 25% of the time. In the example shown in FIG. 6C, a curve 630 shows the measured glucose levels within 24 hours, and a pie chart 632 shows the statistics of the measured glucose levels during the 24 hours. Curve 630 and pie chart 632 show that, within the 24 hours, the measured glucose levels in the illustrated example are within the range of 70-180 mg/dL during about 40% of the time, are below 70 mg/dL during about 20 % of the time, and are above 180 mg/dL during about 40% of the time.

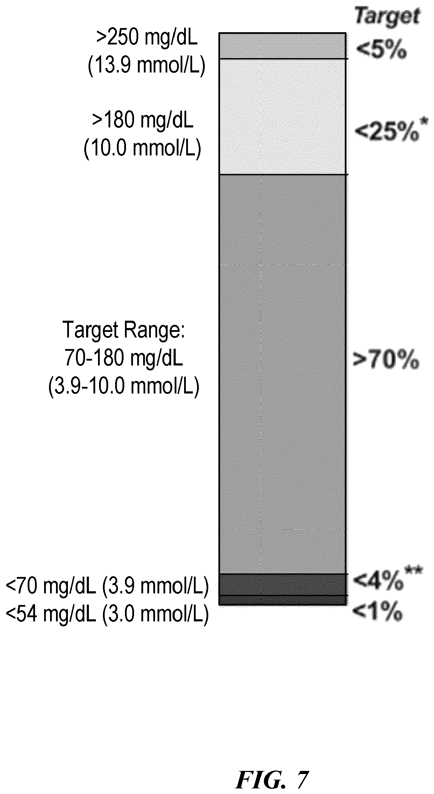

FIG. 7 illustrates an example of the recommended targets of a time-in-range metric for evaluating a glycemic control system. In the example shown in FIG. 7, the recommended TIR targets for glycemic control for Type 1 diabetes patients may include: over 70% of time within a range of 70-180 mg/dL (or about 3.9-10.0 mmol/L) (TIR), less than 25% of time above 180 mg/dL (or 10.0 mmol/L) (TA180), less than 5% of time above 250 mg/dL (or 13.9 mmol/L) (TA250), less than 4% of time below 70 mg/dL (or about 3.9 mmol/L) (TB70), and less than 1% of time below 54 mg/dL (or 3.0 mmol/L) (TB54). There is also a growing interest in a newly proposed metric, the percentage of time in a tight glucose range of 70-140 mg/dL (TITR), as a potential indicator of optimal CGM euglycemia.

Automated insulin delivery (AID) systems, such as Medtronic MiniMedTM 780G, have shown good performance in glycemic control, including the feasibility of achieving GMI targets close to 6.5% or lower. However, in many glycemic control systems, there may need to be some trade-offs in achieving or exceeding these recommended targets as efforts to improve one glycemic metric may have mixed effects on other CGM glycemic metrics. For example, increasing insulin doses may lower the GMI, TA180, and TA250, but may potentially increase the TB70 and TB54. Therefore, it may be desirable to determine whether meeting a specific glycemic target (e.g., a certain GMI target) may correspond to the compliance with certain targets of other glycemic metrics, such as the TIR, TITR, TB70, TB54, TA180, or TA250 targets, such that appropriate targets may be expected by the patients and/or may be used to control the glycemic control systems (e.g., an AID system) to achieve the desired results for specific patients. In addition, it may be desirable to explore the dimensionality of these CGM metrics to establish implications of, for example, targets of the time in various ranges based on GMI targets.

According to certain embodiments, techniques for determining the corresponding targets of one or more CGM metrics based on the target of another CGM metric are disclosed. In one example, a classifier may be trained to classify CGM data samples, and the classification results of the classifier may be used to determine whether a performance of a glycemic control system meeting a certain target of a first glycemic metric (e.g., GMI) can correspond to a performance meeting a threshold target of a second glycemic metric (e.g., TIR). For example, for a given target of the first glycemic metric (e.g., GMI less than 7%, less than 6.8%, less than 6.6%, or less than 6.5%), the classification results for different threshold target levels of the second glycemic metric (e.g., TIR, TITR, TB70, TB54, TA180, or TA250) may be determined and used to generate a receiver operating characteristic (ROC) curve. Based on the ROC curve, an appropriate threshold target level of the second glycemic metric that may maximize both the sensitivity and specificity of the classification (e.g., the threshold target level associated with a point on the ROC curve that is closest to the top-left corner) may be selected. The selected threshold target level of the second glycemic metric that may maximize both the sensitivity and specificity of the classification may be used as the target of the second glycemic metric that may be associated with or correspond to the target of the first glycemic metric. In this way, corresponding target values and possible trade-offs between two or more different CGM metrics may be determined and used for setting patient expectations and setting the control targets of glycemic control systems. The target of the second glycemic metric can be for a population or may be personalized.

In addition, dimension or parameter reduction techniques (e.g., principal component analysis (PCA)) and correlation techniques (e.g., pairwise Pearson correlation) may be used to determine the association between the GMI and the TIR metrics based on the CGM data samples, and reduce the dimensionality of these CGM metrics while preserving relevant information (e.g., majority of the variance) inherent in the CGM data samples. For example, the TIR metrics and the GMI metric may be clustered to identify metrics that are associated with GMI, such as belonging to the same dimension or the orthogonal dimension with respect to GMI, thereby validating the determination of targets for time in various ranges associated with the GMI targets.

In one example, real-world data from individuals with Type 1 diabetes and treated with Medtronic MiniMedTM 780G systems was collected and used to determine the appropriate targets for TIR, TITR, TB70, TB54, TA180, and TA250 that may correspond to GMI targets less than about 7%, less than about 6.8%, less than about 6.6%, and less than the 6.5%, using artificial intelligence and machine learning techniques. For example, the data may be used to predict whether achieving a GMI target of, for example, less than 7% or another value, may guarantee the compliance with consensus targets for TIR, TB70, TB54, TA180, and TA250. The data has also been analyzed to identify and quantify the trade-offs in glycemic control when pursuing stricter GMI benchmarks. The data has also been used to determine the association and dimensionality of these CGM metrics and establish implications of CGM targets based on GMI targets. The results indicate that targets for time in hypoglycemia (e.g., TB70 and TB54) may not be reliably stablished based on GMI targets, but targets for TB54 may be reliably stablished based on targets for TB70. The results may be used for decision making on treatment goals and for setting achievable CGM glucose metrics, which may be used in setting realistic expectations for diabetes management using AID systems.

The data used in the example includes anonymous real-world data uploaded from Medtronic MiniMedTM 780G systems to Medtronic CareLinkTM during a time period longer than 2 years, including data for 14,459 individuals with ages equal to or less than 15 and 41,692 individuals with ages greater than 15. Medtronic MiniMedTM 780G system may operate in an open-loop mode, or an advanced hybrid closed loop (AHCL or automatic) mode in which the current and past glucose levels measured by a CGM sensor and the trends of the glucose level may be used to anticipate, adjust, and correct insulin delivery. The 56,151 individuals include users from different countries. Each individual of the 56,151 individuals has Type 1 diabetes (TID), has a registered CareLinkTM personal account, and has assented to the use of the data for research. Each individual has 10 or more days of sensor glucose (SG) data after the AHCL was enabled for the first time. All available CGM data from all eligible users (analysis cohort) were used, regardless of whether the system was in the AHCL mode or the open-loop mode.

The CGM data may be divided into a plurality of CGM data samples. For example, each CGM data sample may include CGM data of a user during a period of time (e.g., about 10-14 days or longer). The GMI and TIR metrics for the plurality of CGM data samples may be determined and used as training samples and/or test samples. To avoid overfitting in the identification of the appropriate targets and to perform an unbiased evaluation, the CGM data may be divided into a training dataset (e.g., random CGM data samples for about 70% of the users) and a test dataset (e.g., CGM data samples for the remaining 30% of the users). In one example, the training dataset may be used to determine the appropriate CGM targets, and the test dataset may be used to independently evaluate the sensitivity, specificity, accuracy, and other performance parameters of the classifiers and the determined targets. In some examples, confidence intervals for the determined targets and the area under the ROC curve (AUC) may be calculated using bootstrap samples from the training dataset.

In the example, among the 14,459 individuals with ages equal to or less than 15, 46.8%, 53.0%, and 0.2% of the individuals reported themselves as male, female, and non-disclosed, respectively. Among the 41,692 individuals with ages greater than 15, 42.8%, 57.0%, and 0.2% of the individuals reported themselves as male, female, and non-disclosed, respectively. Data for the 14,459 individuals with ages equal to or less than 15 is divided into a training dataset (including data for 10,121 users) and a test dataset (including data for 4,338 users). The median duration of using the Medtronic MiniMedTM 780G system by these 14,459 users is about 219 days (with the 25th percentile at 96 days, and the 75th percentile at 391 days). The 41,692 individuals with ages greater than 15 include 27.6% of users with ages between 16-28, 29.9% of users with ages between 29-42, 25.5% of users with ages between 43-55 years, 16.9% of user with ages greater than 56 years. Data for the 41,692 users are divided into a training dataset (including data for 29,184 users) and a test dataset (including data for 12,508 users). The median duration of using the Medtronic MiniMedTM 780G system by the 41,692 users with ages greater than 15 is 179 days (with the 25th percentile at 77 days, and the 75th percentile at 335 days).

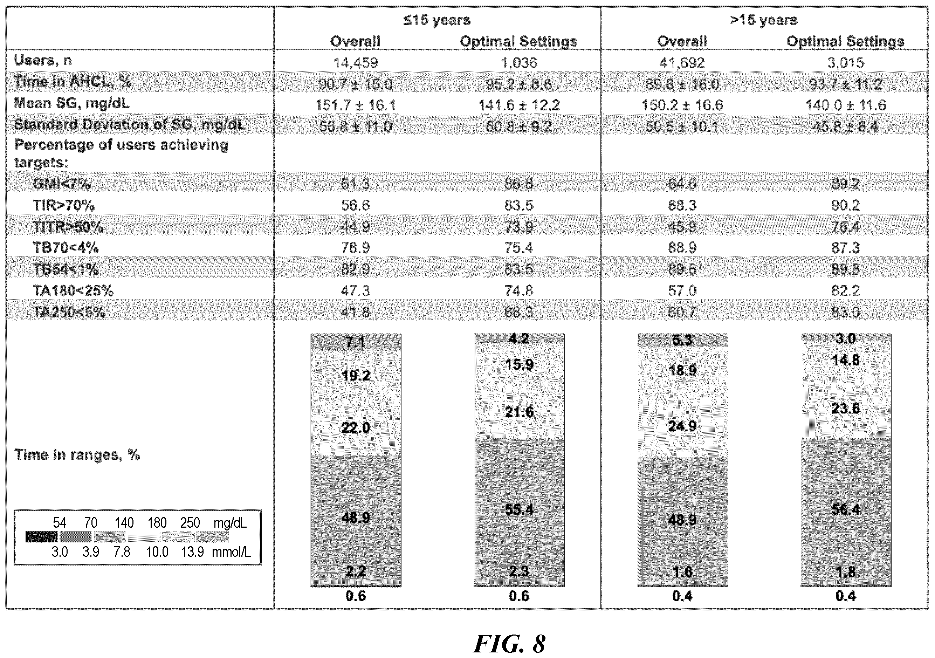

FIG. 8 shows a summary of the above-described example of CGM dataset used to determine the target of a CGM metric based on the target of another CGM metric according to certain embodiments. FIG. 8 shows the statistical data of the total time in the AHCL mode, the sensor glucose (SG) values (including mean and standard deviation (mean±STD)), the percentage of users achieving each of various glucose targets (e.g., GMI < 7%, TIR > 70%, TITR > 50%, TB70 < 4%, TB54 < 1%, TA 180 < 25%, and TA250 < 5%), and the average percentage of time in each of several SG ranges, for the group of 14,459 individuals with ages equal to or less than 15 and the group of 41,692 users older than 15.

FIG. 8 also shows a similar summary of the data for users that consistently adhered to a recommended setting for maintaining a glucose target of 100 mg/dL and an active insulin time (AIT) of 2 hours for over 95% of the time. For example, for users that are 15 years old or younger and consistently use the recommended setting, the mean±STD values for TIR, TITR, TB70, TB54, TA180, and TA250 metrics are 76.9±7.5%, 55.4±8.4%, 2.9±2.0%, 0.6±0.6%, 20.1±7.7%, and 4.2±3.3%, respectively. For users that are older than 15 and consistently used the recommended setting, the mean±STD values for TIR, TITR, TB70, TB54, TA180, and TA250 metrics are 80.0±7.4%, 56.4±9.0%, 2.2±1.7%, 0.4±0.5%, 17.8±7.7%, and 3.0±2.7%, respectively.

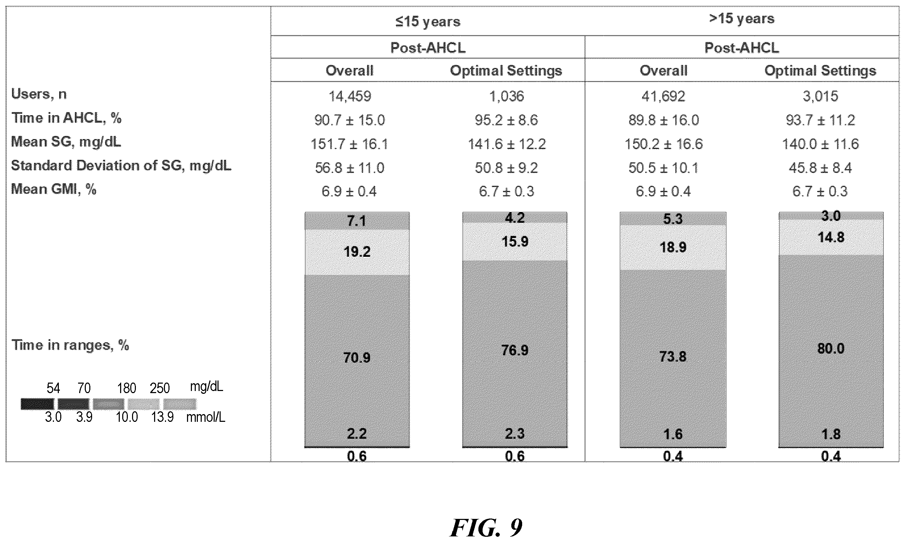

FIG. 9 shows another summary of the example of CGM dataset used to determine the target of a CGM metric based on the target of another CGM metric according to certain embodiments. FIG. 9 shows the statistical data of the total time in the AHCL mode, the sensor glucose (SG) values (including mean and standard deviation), the mean GMI, and the average percentage of time in each of several SG ranges, for the group of 14,459 individuals with ages equal to or less than 15 and the group of 41,692 users older than 15. FIG. 9 shows that, for all users, the mean±STD value of the GMI may be about 6.9±0.4.

FIG. 9 also shows a similar summary of the data for users that consistently adhered to a recommended setting for maintaining a glucose target of 100 mg/dL and an active insulin time (AIT) of 2 hours for over 95% of the time. FIG. 9 shows that, for users that consistently adhered to the recommended setting, the mean±STD value of the GMI may be about 6.7±0.3.

To predict the appropriate targets for TIR, TITR, TB70, TB54, TA180, and TA250 that are associated with targets of GMI less than 7%, less than 6.8%, less than 6.6%, and less than 6.5%, a plurality of classifiers may be trained using the training samples described above to predict whether a CGM data sample meeting a specific GMI target (e.g., 7%, 6.8%, 6.6%, or 6.5%) may also meet a target of a time-in-range metric. The plurality of classifiers may include binary classifiers such as logistic regression models, support vector machines, neural networks, decision trees, and the like. The binary classifiers can classify each CGM data sample meeting a specific GMI target into one of two groups, such as having a time-in-range metric value above or below a threshold value. For example, one classifier of the plurality of classifiers may be used to classify a CGM data samples having a GMI less than 7% as having a TIR greater than a certain threshold value (e.g., 70%) (e.g., a positive result) or having a TIR lower than the threshold value (e.g., a negative result). Another classifier of the plurality of classifiers may be used to classify a CGM data sample having a GMI less than 7% as having a TA180 lower than a certain threshold value (e.g., 25%) (e.g., a positive result) or having a TA180 greater than the threshold value (e.g., a negative result). Yet another classifier of the plurality of classifiers may be used to classify a CGM data sample having a GMI less than 6.8% as having a TIR greater than a certain threshold value (e.g., 70%) (e.g., a positive result) or having a TIR lower than the threshold value (e.g., a negative result).

The classification result of a classifier using a threshold value may be compared with the known TIR metric value of the CGM data sample to determine whether the classification result is a true positive (TP), a false positive (FP), a true negative (TN), or a false negative (FN). In this way, the performance of the classifier, such as the accuracy, true positive rate, true negative rate, false positive rate, and false negative rate of the classifier may be determined for a specific threshold value, using the training samples. The accuracy may be a ratio between the total number of correct predictions and the total number of predictions: accuracy = (TP+TN)/(TP+FP+TN+FN). The true positive rate (TPR, sensitivity, or recall) may be a ratio between the number of true positive predictions and the total number of true positive predictions and false negative predictions (e.g., TPR = TP / (TP + FN)). The false positive rate (FPR) may be a ratio between the number of false positive predictions and the total number of false positive predictions and true negative predictions (e.g., FPR = FP / (FP + TN)). The true negative rate (TNR or specificity) may be a ratio between the number of true negative predictions and the total number of true negative predictions and false positive predictions (e.g., TNR = TN / (TN + FP)). The false negative rate (FNR) may be a ratio between the number of false negative predictions and the total number of false negative predictions and true positive predictions (e.g., FNR = FN / (FN + TP)). For each classifier, the true positive rate and the false positive rate for each threshold value of a plurality of threshold values may be determined as described above, and the true positive rates and the corresponding false positive rates for the plurality of threshold values may be plotted in a receiver operating characteristic (ROC) curve.

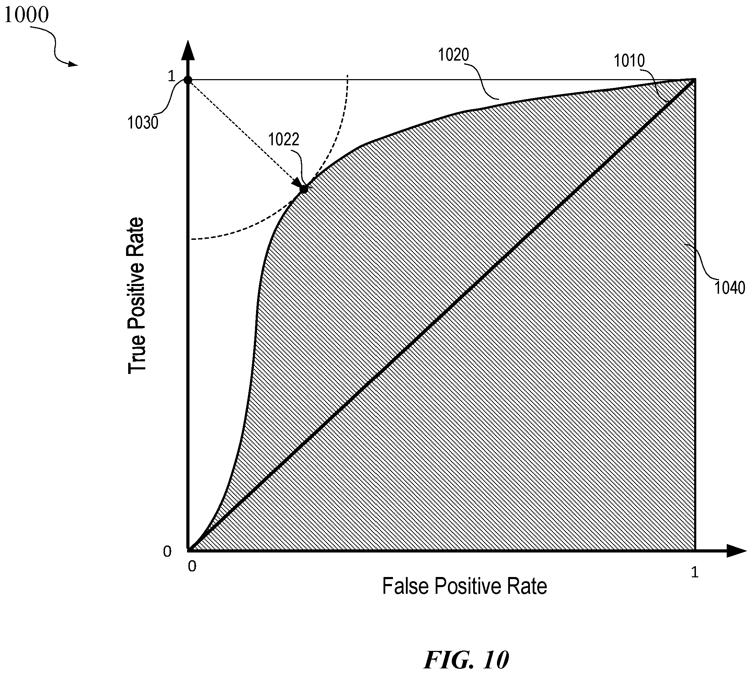

FIG. 10 includes a diagram 1000 illustrating an example of a receiver operating characteristics (ROC) curve 1020 that shows an example of the performance of a classifier for determining the target of a CGM metric based on the target of another CGM metric using various classification thresholds. The horizontal axis of diagram 1000 may correspond to the false positive rate, and the vertical axis of diagram 1000 may correspond to the true positive rate. The true positive rate (sensitivity or recall) represents the proportion of samples that are predicted to be positive when they are indeed positive. The false positive rate represents the proportion of samples that are predicted to be positive when they are actually negative. Each ROC curve is a graph displaying the performance of a binary classification model at every classification threshold. Each point on an ROC curve indicates the TPR and FPR at each respective classification threshold. Both TPR and FPR may increase when the classification threshold is lowered. A line 1010 in diagram 1000 is an ROC of a random classifier. A point 1030 at the top left corner represents an ideal classifier with a TPR of 1 and an FPR of 0. The performance of a classifier may be better if the corresponding ROC curve is closer to the top left corner. The performance of the classifier may be determined based on, for example, the total area under the ROC curve (AUC). The performance of the classifier may be better if the corresponding AUC of the classifier is close to 1.

In the example shown in FIG. 10, ROC curve 1020 is an example of an ROC curve of a classifier described above, such as a classifier that classifies CGM data samples having known GMI results (> 7% or ≤ 7%) into groups meeting or not meeting a target value of a TIR metric (e.g., TIR, TITR, TB70, TB54, TA180, or TA250). An area 1040 shows the AUC of the classifier. To determine an appropriate classification threshold, a point 1022 that is closest to point 1030 among all points on ROC curve 1020 may be determined, and the corresponding classification threshold of point 1022 may be determined as the corresponding target value of the TIR metric that may correspond to the target of GMI metric (e.g., about 7%). The target values of other CGM metrics that correspond to a certain target value of a CGM metric may be determined in the same manner based on the ROC curves of the corresponding classifiers.

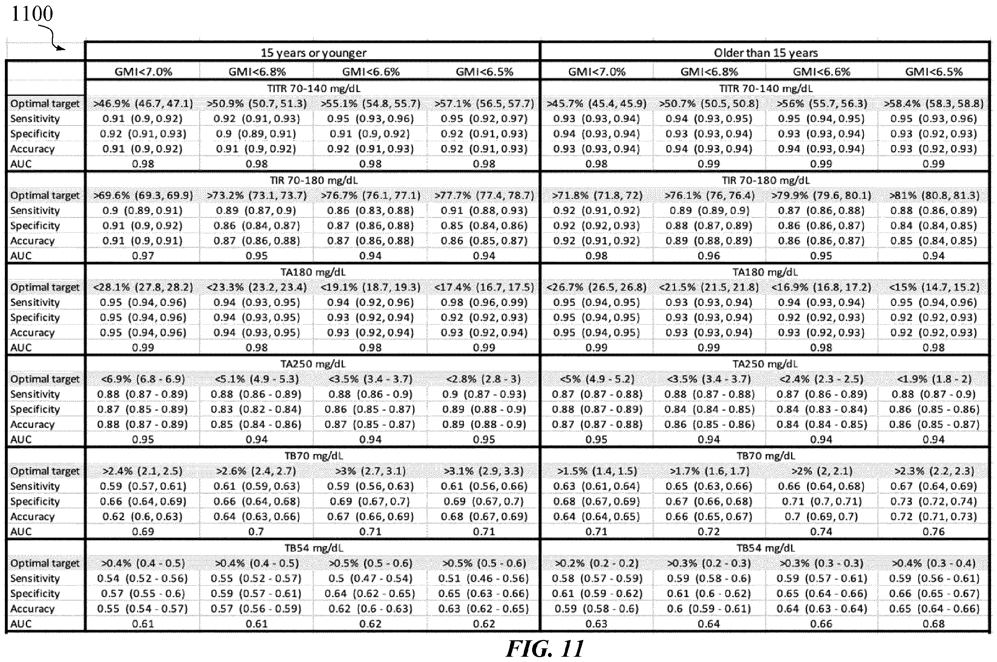

FIG. 11 includes a table 1100 illustrating examples of the determined target values of certain time-in-range metrics that may correspond to different GMI target values according to certain embodiments. The time-in-range metrics may include, for example, TITR, TIR, TA180, TA250, TB70, TB54, and the like. The different GMI target values may include, for example, lower than 7%, lower than 6.8%, lower than 6.6%, lower than 6.5%, and the like. Table 1100 also shows the AUC of the corresponding classifier and the corresponding sensitivity, specificity, and accuracy of the classifier at the determined target values of the time-in-range metrics. Results based on both CGM data samples from users that are 15 years old or younger and CGM data samples from users that are older than 15 are shown in table 1100.

In the illustrated examples, for users that are 15 years old or younger, when the GMI target is lower than 7%, the determined target values for TITR (within 70-140 mg/dL), TIR (within 70-180 mg/dL), TA180 (> 180 mg/dL), TA250 (> 250 mg/dL), TB70 (<70 mg/dL), and TB54 (< 54 mg/dL) are approximately greater than 46.9%, greater than 69.6%, less than 28.1%, less than 6.9%, greater than 2.4%, and greater than 0.4%, respectively. The sensitivity, specificity, and accuracy for the determined target values of TITR, TIR, TA180, and TA250 are relatively high (e.g., about 90% or higher), but the sensitivity, specificity, and accuracy for the determined target values of TB70 and TB54 are relatively low (e.g., between about 50% and about 70%). This may indicate stronger association between the GMI metric and the TITR, TIR, TA180, and TA250 metrics, and weaker association between the GMI metric and the TB70 and TB54 metrics. When the metrics (e.g., TB70 and TB54) have weak association with the GMI metric, identifying reliable targets for the metrics may be challenging.

For other GMI target values (e.g., < 6.8%, < 6.6%, or < 6.5%), the sensitivity, specificity, and accuracy for the determined target values of TITR, TIR, TA180, and TA250 are also relatively high (e.g., about 90% or higher), but the sensitivity, specificity, and accuracy for the determined target values of TB70 and TB54 are relatively low (e.g., between about 50% and about 70%). In particular, the sensitivity, specificity, and accuracy for the determined target values of TITR and TA180 are consistently greater than 90% for all GMI target values, and the AUC of the classifiers for classifying the TITR and TA180 metrics based on the GMI target values may be close to 1 (e.g., $\geq$ 98%) for all GMI target values. For TIR and TA250, the sensitivity, specificity, and accuracy may be slightly lower. This may suggest that TITR or TA180 may be a better metric in glucose management when the goal is to achieve euglycemia.

Similarly, for users that are older than 15, when the GMI target is lower than 7%, the determined target values for TITR, TIR, TA180, TA250, TB70, and TB54 are approximately greater than 45.7%, greater than 71.8%, less than 26.7%, less than 5%, greater than 1.5%, and greater than 0.2%, respectively. The sensitivity, specificity, and accuracy for the determined target values of TITR, TIR, TA180, and TA250 are relatively high (e.g., about 90% or higher), but the sensitivity, specificity, and accuracy for the determined target values of TB70 and TB54 are relatively low (e.g., between about 50% and about 70%). For other GMI target values (e.g., < 6.8%, < 6.6%, or < 6.5%), the sensitivity, specificity, and accuracy for the determined target values of TITR, TIR, TA180, and TA250 are also relatively high (e.g., about 90% or higher), but the sensitivity, specificity, and accuracy for the determined target values of TB70 and TB54 are relatively low (e.g., between about 55% and about 70%). In particular, the sensitivity, specificity, and accuracy for the determined target values of TITR and TA180 are consistently greater than 90% for all GMI target values, and the AUC of the classifiers for classifying the TITR and TA180 metrics based on the GMI target values may be close to 1 (e.g., $\geq$ 98%) for all GMI target values. For TIR and TA250, the sensitivity, specificity, and accuracy may be slightly lower.

FIG. 11 also shows that, as the GMI target became more stringent (e.g., progressing from <7% to <6.5%), the reliability of the determined time-in-range targets in association with the GMI target may slightly decrease, as evidenced by the decrease of the values of sensitivity, specificity, accuracy (e.g., decreasing from about 90-92% to about 86-84%), and AUC (e.g., decreasing from about 97-98% to about 94%). In contrast, the reliability of the determined TITR targets associated with the different GMI targets may be consistently high, regardless of the stringency of the GMI targets.

As also illustrated by FIG. 11, the determined targets for TB70 and TB54 may have the lowest values for sensitivity, specificity, accuracy (e.g., as low as 50%), and AUC (e.g., as low as 61%), which may suggest that the targets for time spent in hypoglycemic ranges may be less consistently associated with the GMI targets. Further investigation into this discrepancy is described in more detail below.

The results in table 1100 show that, for the target of GMI less than 7%, the corresponding TIR target may be greater than about 70% for users 15 years old or younger, and may be greater than about 72% for users older than 15. For the same target of GMI less than 7%, the corresponding TA180 target may be less than about 28% for users 15 years old or younger and less than about 27% for users older than 15, whereas the corresponding TA250 target may be less than about 7% for users 15 years old or younger and less than about 5% for users older than 15. Even though the consensus limits for TA180 and TA250 are set to <25% and <5%, respectively, the results shown in table 1100 indicate that a TA180 target between about 28% and 25% (for users 15 years old or younger) or between about 26.7% and 25% (for users older than 15) may correspond to a GMI target less than 7%, while a TA250 target between about 6.9% and 5% (for users 15 years old or younger) or about 5% or lower (for users older than 15) may correspond to a GMI target less than 7%. In general, these target values closely align with the recommended international consensus targets for time-in-range metrics described above, which indicates that the recommended consensus targets for the GMI metric and the time in various ranges metrics for Type 1 diabetes are generally consistent and accurately jointly defined.

The results in table 1100 further show that, for the target of GMI less than 7%, the associated TITR target may be greater than about 47% for users 15 years old or younger, and may be greater than about 46% for users older than 15. The associated TITR target may be greater than about 51% for the target of GMI less than 6.8% for both age groups. As shown in FIG. 8, the TITR target of greater than about 50% may be achieved by about 45% of the users 15 years old or younger, and by about 46% of the users older than 15, which indicates that a TITR target of greater than about 50% may be reasonable for both younger and older users.

The data used in the analysis also shows that Medtronic MiniMed™ 780G systems can achieve a mean TB70 value and a mean TB54 value well within the clinical targets of <4% and <1%, respectively, as shown in FIGS. 8 and 9. FIGS. 8 and 9 also show that lower mean GMI and larger mean TIR and mean TITR can be achieved with the consistent use of the recommended settings, while maintaining TB70 and TB54 well below the recommended targets (e.g., a mean TB70 about 2.9% and 2.4% for the young and the older users, respectively, and a mean TB54 about 0.6% and 0.4% for the young and the older users, respectively). However, the results in table 1100 indicate that a mean TB70 less than 2% may be less likely for the target of GMI lower than about 6.5.

The results in table 1100 also quantify the relationships or trade-offs between meeting or exceeding the targets for various CGM metrics of glycemic control. For example, attempting to achieve a low mean GMI may be accompanied by a decrease in time in hyperglycemia and an increase in TIR and TITR. In one example, for users older than 15, when the target of the GMI metric is less than 6.6%, the corresponding TIR target may be greater than 80%, the corresponding TITR target may be greater than 56%, the corresponding TB70 target may be greater than 2%, and the corresponding TA180 target may be lower than 17%.

Therefore, techniques and results disclosed herein may be utilized to formulate a treatment strategy to improve TIR and TITR and reduce time in hypoglycemia. For example, to maintain a mean TB70 less than 3%, the achievable mean TIR may be larger than 70% but would likely not exceed 78% (or a TITR greater than 55%) for users 15 years old or younger. A TIR greater than 78% may be possible, but may likely be accompanied by a TB70 more than 3% (still below the recommended target of <4%). For users older than 15, a TIR of 81% (or a TITR of 58%) may be achievable, at a cost of a TB70 greater than 2.3%. In general, the trade-offs are seen in both age groups, but the older age group may be more likely to reach tighter glycemic goals. These techniques and results may enable health care providers and people living with Type 1 diabetes to establish realistic expectations regarding the overall glycemic control performance, when pursuing more stringent targets of time in various ranges and GMI metrics using AID systems.

The examples described above show techniques and results of determining the target of a CGM metric (e.g., a time-in-range metric or GMI metric) for a population based on the target of another CGM metric (e.g., another time-in-range metric or GMI metric) and population CGM data samples associated with a specific glycemic control system such as Medtronic MiniMed™ 780G. Similar techniques may be used to determine the target of a CGM metric for a population based on the target of another CGM metric and/or population CGM data sample associated with another glycemic control system. Similar techniques may also be used to determine the target of a CGM metric for a specific user based on the target of another CGM metric and personal CGM data samples associated with the specific glycemic control system. For example, CGM data for a user of the specific glycemic control system may be collected over a time period and divided into a plurality of sections (e.g., each including data for 10-14 days or longer) corresponding to a plurality of CGM data samples, and the GMI and TIR metrics of each CGM data sample may be determined and used as a training sample or a test sample for determine the target of a CGM metric for the user based on the target of another CGM metric using techniques described above.

As described above, the results shown in, for example, FIG. 11, indicate that there may be stronger association between some CGM metrics and weaker association between some CGM metrics. To determine the meaningful targets for time-in-range metrics according to the GMI target, it may be desirable to determine the association between GMI and the time-in-range metrics, such as TIR, TITR, TB70, TB54, TA180, and TA250. According to certain embodiments, dimension or parameter reduction techniques (e.g., principal component analysis (PCA)) and correlation techniques (e.g., pairwise Pearson correlation) may be used to determine the association between the GMI and the various time-in-range metrics based on the CGM data samples described above, and reduce the dimensionality of these CGM metrics while preserving relevant information (e.g., majority of the variance) inherent in the CGM data samples. For example, the time-in-range metrics and the GMI metric may be clustered to identify metrics that are associated with GMI, such as belonging to the same dimension or the orthogonal dimension with respect to GMI, thereby validating the determination of targets for time in various ranges associated with the GMI targets.

The Pearson correlation coefficient may be used to measure a linear correlation between two variables, and may have a value between -1 and 1 that measures the strength and direction of the relationship between the two variables. A Pearson correlation coefficient close to zero indicates that there is no or weak relationship between the two variables. A Pearson correlation coefficient between 0 and 1 indicates a positive correlation, where a change in one variable may cause a change of the other variable in the same direction. A Pearson correlation coefficient between 0 and -1 indicates a negative correlation, where a change in one variable may cause a change of the other variable in the opposite direction.

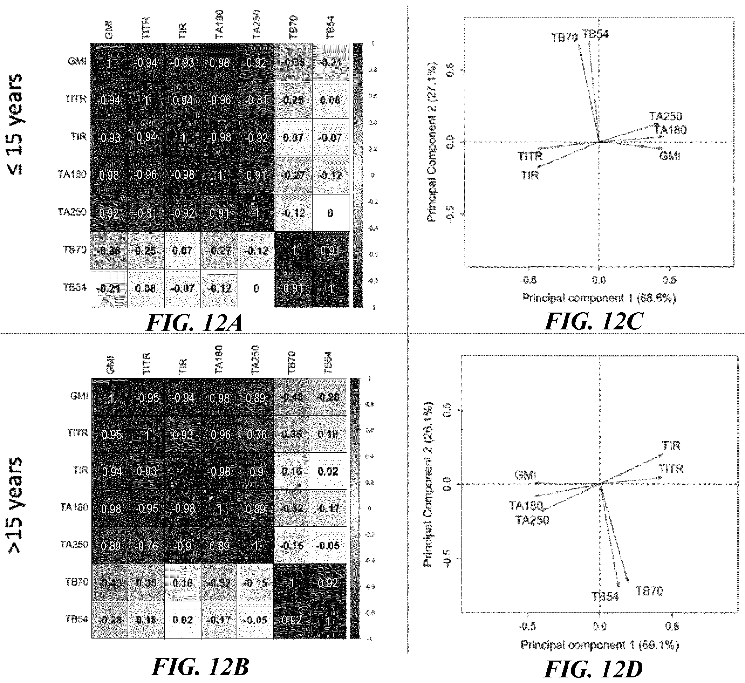

FIGS. 12A and 12B show results of examples of Pearson correlation analysis of six time-in-range metrics and the GMI metric for users 15 years old or younger and users older than 15, respectively. In FIGS. 12A and 12B, the darker boxes indicate stronger positive or negative pairwise correlation. The value in each box indicates the corresponding Pearson correlation coefficient. A larger positive Pearson correlation coefficient (e.g., closer to 1, such as 0.98 between GMI and TA180) indicates a stronger positive correlation, while a smaller negative Pearson correlation coefficient (e.g., closer to -1, such as -0.98 or -0.99 between TIR and TA180) indicates a stronger negative correlation. A Pearson correlation coefficient closer to zero (e.g., about 0 or -0.05 between TA250 and TB54, or about -0.07 or 0.02 between TIR and TB54) indicates weak or no correlation. The results in FIGS. 12A and 12B show two distinct groups of CGM metrics for both age groups, where the metrics in each group may have high within-group correlation and low between-group correlation for both age groups. For example, the metrics of GMI, TITR, TIR, TA180, and TA250 may have strong correlation between each other, but may have weak correlation with the TB70 and TB54 metrics, while TB70 and TB54 metrics may have a strong correlation. In the first group, the TITR, TIR, TA180, and TA250 metrics all exhibit strong correlation with GMI (e.g., with absolute correlation >0.89). The second group includes TB70 and TB54 metrics associated with hypoglycemia. Each of TB70 and TB54 metrics may have a lower correlation with GMI (e.g., with absolute correlation <0.43), which indicates that the metrics for time spent in hypoglycemic ranges may not align in the same dimension as the GMI metric.

PCA is a statistical technique in the field of multivariate data analysis, aiming to explain the most variance using the fewest components. PCA can be used herein to reduce the dimensionality and complexity of these CGM metrics while preserving relevant information (e.g., majority of the variance) in the dataset. Using the PCA technique, time-in-range metrics that align with the GMI metric within the same principal component and therefore are part of the same dimension may be identified. The identification of the time-in-range metrics that align with the GMI metric can highlight the time-in-range metrics that are closely associated with the GMI metric, and justify the time-in-range target determination based on the GMI targets described above. For time-in-range metrics that are in a different (e.g., orthogonal) dimension from the GMI metric, the targets may need to be set based on targets of other pertinent CGM metrics that are in the same dimension as such time-in-range metrics.

FIGS. 12C and 12D show results of PCA analysis of six time-in-range metrics and the GMI metric for users 15 years old or younger and users older than 15, respectively. In the examples shown in FIGS. 12C and 12D, the dimensionality of these CGM metrics is reduced to two using the Kaiser criterion guideline, where the horizontal axis represents a first principal component that explains the most variance, and the vertical axis represents a second principal component that explains the second most variance. Each principal component may be a new, transformed variable that is a linear combination of the original variables. Each vector in FIGS. 12C and 12D indicates the contribution (or load) of a glycemic metric to the two principal components or dimensions. FIGS. 12C and 12D show that, for both age groups, TITR, TIR, TA180, and TA250 metrics align closely with the GMI metric within the first principal component. The second principal component is primarily composed of TB70 and TB54, the hypoglycemia metrics.

FIG. 12C shows that the two dimensions or principal components explain most (e.g., about 95.7%) of the total variance for users 15 or younger, where the first principal component explains about 68.6% of the variance and the second principal component explains about 27.1% of the variance. FIG. 12D shows that the two principal components explain about 95.2% of the total variance for users older than 15, where the first principal component explains about 69.1% of the variance and the second principal component explains about 26.1% of the variance.

The Pearson correlation and PCA analysis described above consistently place TITR, TIT, TA180, and TA250 within the same dimension as GMI, and place TB70 and TR54 in a different dimension from GMI. As shown in table 1100 of FIG. 11 described above, the lowest sensitivity, specificity, accuracy, and AUC may be associated with the TB70 and TB54 targets for any given GMI target, indicating weak association between the time in hypoglycemic range metrics and the GMI metric. The results of the Pearson correlation and PCA analysis confirm that targets for TIR, TITR, TA180, and TA250 may be reliably determined based on the GMI targets.

The results of the Pearson correlation and PCA analyses described above further confirm that the TB70 and TB54 metrics measure a dimension of glycemic control related to hypoglycemia, which the GMI metric does not align in. This indicates that targets for these hypoglycemia metrics may not be reliably determined based solely on the GMI targets. Therefore, tighter glycemic control (e.g., lower GMI or HbA1c) may not necessarily imply an increase in time below range (TBR), and reducing TBR may not necessarily lead to lower TIR or higher GMI. For example, reducing the time above a range (TAR) may improve GMI, and reducing TBR may be achieved without worsening(increasing) GMI. Since TB70 and TB54 metrics align in the same dimension, it may be possible to reliably estimate the targets of one of TB54 and TB70 based on the target of the other one of TB54 and TB70.

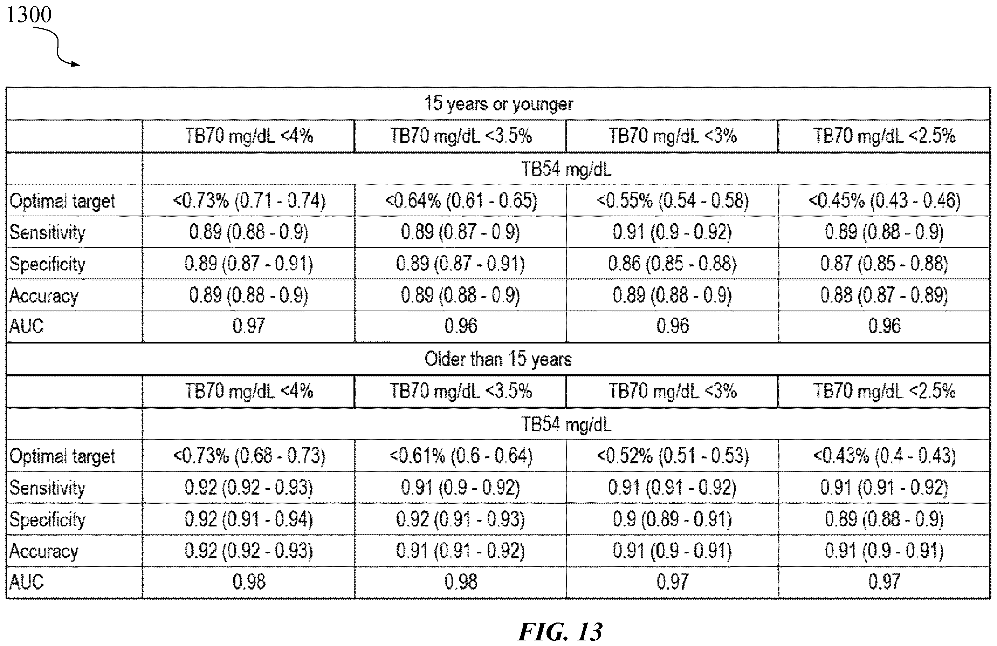

FIG. 13 includes a table 1300 showing examples of the results of estimating targets of TB54 associated with various TB70 targets according to certain embodiments. The results in FIG. 13 may be determined using techniques similar to the techniques for determining the results shown in FIG. 11. In table 1300, the targets of the TB70 metric used for the TB54 target estimation include lower than 4%, lower than 3.5%%, lower than 3%, and lower than 2.5%. FIG. 13 shows that a TB70 target of less than 4% may correspond to a TB54 target of less than 0.73% for both age groups, while a TB70 target of lower than 2.5% may correspond to a TB54 target of lower than 0.45% and 0.43% for users 15 or younger and users older than 15, respectively. The AUC for both age groups are relatively high, and the sensitivity, specificity, and accuracy are all better than 85% for both age groups and all four TB70 targets.

The techniques and results described above may be useful in establishing and verifying the propriety of the consensus targets for glycemic control. The results reveal and quantify the trade-offs between targets for various CGM metrics, such that users may know the realistic expectation of other CGM metrics when setting the target of a CGM metric on an AID system. For example, the corresponding targets of the CGM metrics may be determined for an AID system and a population or a specific user as described above, and a user application may be provided to the user to convert a target of a first CGM metric entered or selected by the user to estimated corresponding targets of other CGM targets, such that the user may know the expected performance the AID system in other CGM metrics. If the estimated corresponding target of another CGM metric does not meet the desired glucose control performance, the user may select a different target of the first CGM metrics and determine if the estimated corresponding targets of other CGM metrics meet the desired glucose control performance. In this way, different CGM glycemic metrics may be considered for glycemic control using an AID system. In addition, techniques disclosed herein can be used to provide support and basis for consensus target recommendations for new CGM metrics (e.g., the consensus targets for TITR or another CGM metric), for example, based on universally agreed-upon targets of other CGM metrics (e.g., GMI or TIR).

Techniques disclosed herein may be used to determine the association of different CGM metrics for either a population or a specific user. Techniques disclosed herein may be used to determine the association of different CGM metrics for different AID systems, and may also be applied to applications beyond Type 1 diabetes and AID systems. For example, techniques disclosed herein can also be used to determine the association and dimensionality of CGM metrics using a diverse dataset including data for individuals living with Type 1 diabetes or Type 2 diabetes, individuals receiving multiple daily injections, individuals using CGM alone, individuals using sensor-augmented pump therapy, and the like.

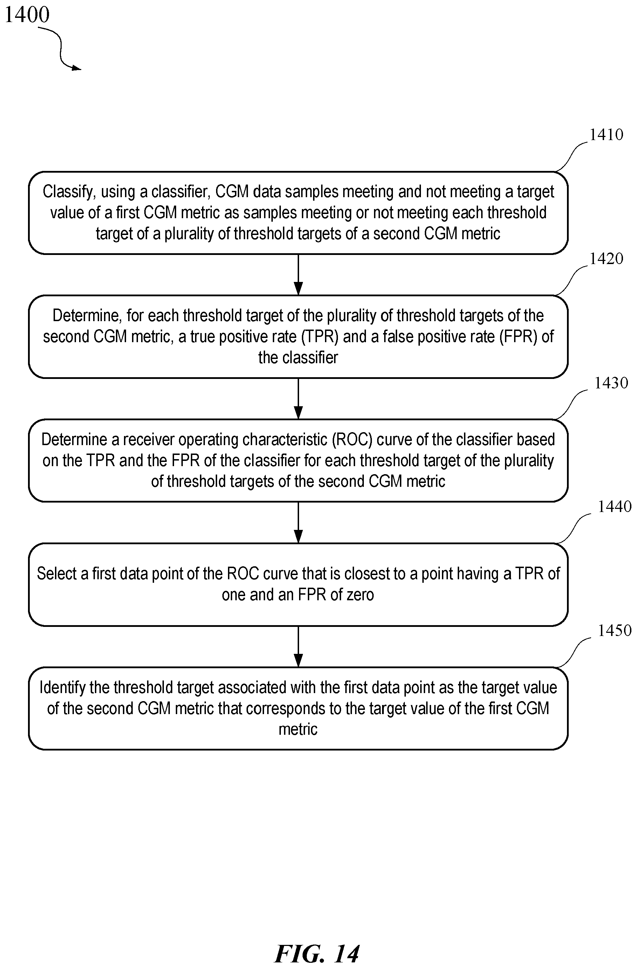

FIG. 14 includes a flowchart 1400 illustrating an example of a method of determining a target value of a CGM metric that corresponds to a target value of another CGM target according to certain embodiments. Operations in flowchart 1400 may be performed using, for example, one or more processors or computing systems (e.g., a server, a computing system 1600 described below, or a smart user device). Although flowchart 1400 may describe the operations as a sequential order, some of the operations may be performed in parallel or concurrently. In addition, the order of the operations may be rearranged. The process may have additional steps not included in the flowchart. Some operations may be optional or may be omitted in some implementations. Some operations may be performed more than one time.

In the example illustrated in FIG. 14, the operations in flowchart 1400 may include, at block 1410, classifying, using a classifier, CGM data samples meeting and not meeting a target value of a first CGM metric as samples meeting or not meeting each threshold target of a plurality of threshold targets of a second CGM metric. As described above, the classifier may be binary classifier, and may include, for example, a logistic regression model, a support vector machine, a neural network, a decision tree, and the like. The CGM data samples may include, for example, CGM data samples of a user or CGM data samples of a population. The CGM data samples may include CGM data samples of users in an age group. In one example, the CGM data samples may include CGM data samples of users of an automatic insulin delivery system. In one example, the CGM data samples may include CGM data samples of users living with Type 1 diabetes and/or users living with Type 2 diabetes. In one example, each CGM data sample may include CGM data of a user during a time period (e.g., 10-14 days or longer). In some examples, CGM metrics for each CGM data sample may be determined and associated with the CGM data sample. In some examples, the CGM data samples may be divided into training samples and test samples. The first CGM metric may include, for example, GMI, TIR, TITR, TA180, TA250, TB70, or TB54. The second CGM metric may include, for example, GMI, TIR, TITR, TA180, TA250, TB70, or TB54. In one example, the target value of the first CGM metric may be a GMI lower than 7%, 6.8%, 6.6%, or 6.5%, and the second CGM metric may include TIR, TITR, TA180, TA250, TB70, or TB54.

Operations in block 1420 may include determining, based on the classification results, a true positive rate (TPR) and a false positive rate (FPR) of the classifier for each threshold target of the plurality of threshold targets of the second CGM metric. The TPR may be a ratio between the number of true positive classification and the total number of true positive classification and false negative classification (e.g., TPR = TP / (TP + FN)). The FPR may be a ratio between the number of false positive classification and the total number of false positive classification and true negative classification (e.g., FPR = FP / (FP + TN)).

Operations in blocks 1430-1450 may include identifying a target value of at least the second CGM metric based on the TPR and the FPR of the classifier for each threshold target of the plurality of threshold targets of the second CGM metric. For example, at block 1430, a receiver operating characteristic (ROC) curve of the classifier may be generated based on the TPR and the FPR of the classifier for each threshold target. Each point of the ROC curve may correspond to a different threshold target of the plurality of threshold targets of the second CGM metric, and may have the corresponding TPR and FPR.

Operations in block 1440 may include selecting a first data point of the ROC curve that is closest to a point having a TPR of one and an FPR of zero. The selection may be based on distances between the top left corner of the ROC graph and points on the ROC curve. The selected data point may have both high TPR and low FPR.

Operations in block 1450 may include identifying the threshold target associated with the first data point as the target value of the second CGM metric that corresponds to the first target value of the first CGM metric.

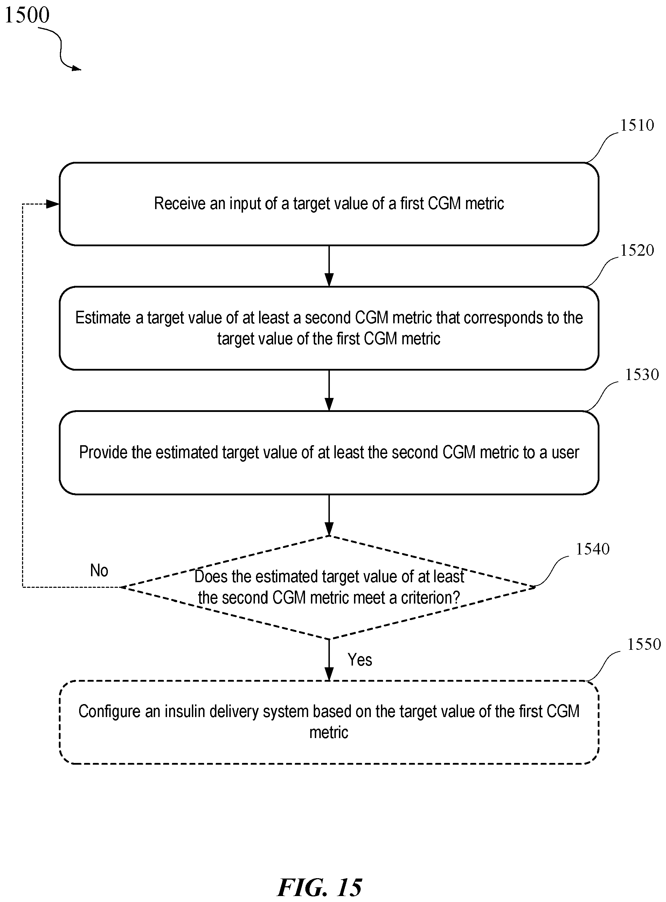

FIG. 15 includes a flowchart 1500 illustrating an example of a processor-implemented method for providing a target value of a CGM metric that corresponds to a target value of another CGM target to a user according to certain embodiments. Operations in flowchart 1500 may be performed using, for example, one or more processors or computing systems (e.g., a server, a computing system 1600 described below, or a smart user device, such as a smartphone). Although flowchart 1500 may describe the operations as a sequential order, some of the operations may be performed in parallel or concurrently. In addition, the order of the operations may be rearranged. The process may have additional steps not included in the flowchart. Some operations may be optional or may be omitted in some implementations. Some operations may be performed more than one time. In some examples, the processor-implemented method may be implemented as a user application that may be executed on a smart user device, such as a smartphone.

In the example illustrated in FIG. 15, the operations in flowchart 1500 may include, at block 1510, receiving an input of a target value of a first continuous glucose monitoring (CGM) metric. As described above, the first CGM metric may include GMI, TIR, TITR, TA180, TA250, TB70, or TB54. For example, the first CGM metric may be GMI, and the target value of the first CGM metric may be lower than 7%, lower than 6.8%, lower than 6.6%, lower than 6.5%, lower than 6.4%, lower than 6.2%, lower than 6%, or even lower.

Operations in block 1520 may include estimating a target value of at least a second CGM metric that corresponds to the target value of the first CGM metric. The method for estimating the target value of at least the second CGM metric that corresponds to the target value of the first CGM metric may be similar to the method described above with respect to FIG. 14.

Operations in block 1530 may include providing the estimated target value of at least the second CGM metric to a user. In some examples, the method may also include determining, for example, based on user feedback, if the estimated target value of at least the second CGM metric meets a predetermined criterion at block 1540; and, at block 1550, configuring an insulin delivery system based on the target value of the first CGM metric if the estimated target value of at least the second CGM metric meets the predetermined criterion. If the estimated target value of at least the second CGM metric does not meet the predetermined criterion, the user may be prompted to enter or select another target value.

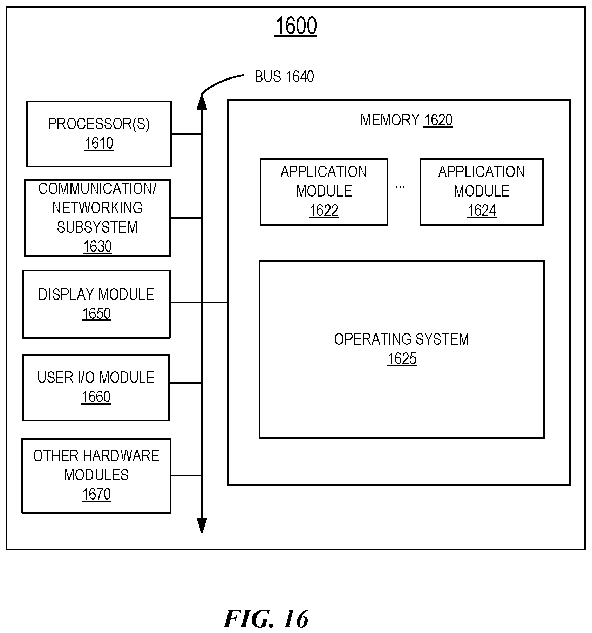

FIG. 16 is a block diagram of an example of an computing system 1600 that may implement some of the examples disclosed herein. For example, computing system 1600 may be used to implement delivery device 102, monitoring device 104, computing device 106, and remote/cloud computing system 108, glucose sensor subsystem 210, controller 220, insulin delivery subsystem 230, glucose delivery subsystem 240, and glucagon delivery subsystem 250, CGM system 300, glucose sensor device 400, delivery device 500, or another device that may be used to calculate insulin dose and/or predict future blood glucose levels. It should be noted that FIG. 16 is meant only to provide a generalized illustration of various components, any or all of which may be utilized as appropriate. In addition, it can be noted that components illustrated by FIG. 16 can be localized to a single device and/or predict future blood glucose levels. In distributed among various networked devices, which may be disposed at different geographical locations.

In the illustrated example, computing system 1600 may include one or more processor(s) 1610 and memory 1620. Processor(s) 1610 may be configured to execute instructions stored in memory 1620 for performing one or more of the methods described herein and other applications. Processor(s) 1610 may include, for example, one or more central processing units, microprocessors, microcontrollers, special-purpose processors (e.g., digital signal processors), ASICs, DSPs, FPGAs, or other processors suitable for implementation within a portable electronic device. Processor(s) 1610 may be communicatively coupled with a plurality of components within computing system 1600. To realize this communicative coupling, processor(s) 1610 may communicate with the other illustrated components across a bus 1640. Bus 1640 may be any subsystem adapted to transfer data within computing system 1600. Bus 1640 may include a plurality of computer buses and additional circuitry to transfer data.

Memory 1620 may include one or more transitory and/or non-transitory storage devices, such as, for example, a static random-access memory (SRAM), a dynamic random access memory (DRAM), a read-access memory (RAM), a read-only memory (ROM), a programmable read-only memory (PROM), an erasable programmable read-only memory (EPROM), a FLASH-EPROM, a secure digital (SD) card, and any other memory chip or cartridge. Such storage devices may be configured to implement any appropriate data stores, including without limitation, various file systems, data structures, computer-readable instructions, program modules, and the like. In some embodiments, memory 1620 may be distributed into different hardware modules. A set of instructions and/or code might be stored on memory 1620. The instructions might take the form of executable code that may be executable by computing system 1600, and/or might take the form of source and/or installable code, which, upon compilation and/or installation on computing system 1600 (e.g., using any of a variety of generally available compilers, installation programs, compression/decompression utilities, etc.), may take the form of executable code.

Memory 1620 may include an operating system 1625 loaded therein. Operating system 1625 may be operable to initiate the execution of the instructions provided by application modules 1622-1824 and/or manage other hardware modules 1670, as well as interface with a communication subsystem 1630 which may include one or more wired and/or wireless transceivers. Operating system 1625 may be adapted to perform other operations across the components of computing system 1600 including threading, virtualization, resource management, data storage control, and other similar functionality. In some embodiments, memory 1620 may store a plurality of application modules 1622 through 1624, which may include any number of applications. Examples of the applications may include an insulin calculator, a blood glucose level monitor or predictor, a glucose level management application, and the like. Application modules 1622-1624 may include particular instructions to be executed by processor(s) 1610. In some embodiments, certain applications or parts of application modules 1622-1624 may be executable by other hardware modules 1670.

Communication subsystem 1630 may include, for example, an infrared communication device, a wireless communication device and/or chipset (such as a Bluetooth® device, an IEEE 802.11 device, a Wi-Fi device, a WiMax device, cellular communication devices, etc.), and/or similar communication interfaces. One or more antennas (not shown) may be used for wireless communication as part of communication subsystem 1630 or as a separate component coupled to any portion of computing system 1600, such as a wireless charging receiver or a near-field communication receiver. In some embodiments, communication subsystem 1630 may include circuits for wired communication technologies, such as Ethernet, coaxial communications, universal serial bus (USB), and the like. In some embodiments, communication subsystem 1630 may include transceivers to communicate with base transceiver stations and other wireless devices and access points, which may include communicating with different data networks and/or network types, such as wide-area networks ("WANs"), wireless wide-area networks (WWANs), local area networks (LANs), wireless local area networks (WLANs), personal area networks (PANs), or wireless personal area networks (WPANs). A WWAN may be, for example, a WiMax (IEEE 802.16) network. A WLAN may be, for example, an IEEE 802.11x network. A WPAN may be, for example, a Bluetooth network, an IEEE 802.15x, or some other types of network. The techniques described herein may also be used for any combination of WAN, LAN, PAN, WWAN, WLAN, and/or WPAN. Communications subsystem 1630 may permit data to be exchanged with a network, other computer systems, and/or any other devices. For example, communications subsystem 1630 may be used to receive therapy determinations for therapeutic fluid (e.g., insulin) delivery, such as from a cloud computing system via an intermediary computing device (e.g., a controller) communicatively coupled to computing system 1600, where processor(s) 1610 may, based on the therapy determinations, send commands to an actuator controller to cause the delivery of appropriate amounts of therapeutic fluid (e.g., insulin) to a user. In another example, communications subsystem 1630 may be used to communicate measurement results (e.g., sensor glucose levels) to a computing device (e.g., a smartphone or a personal health monitoring device) and/or to a remote server via the computing device, or receive data (e.g., calibration data, configuration data, etc.) from the computing device or the remote server via the computing device.

Computing system 1600 may include a display module 1650. Display module 1650 may present information, such as text, images, audios, videos, and various instructions, from computing system 1600 to a user. Such information may be derived from one or more application modules 1622-1624, communication/networking subsystem 1630, one or more other hardware modules 1670, a combination thereof, or any other suitable means. For example, display module 1650 may be used to display user challenges that may include text, images, waveforms, audio clips, video clips, and the like. Display module 1650 may use liquid crystal display (LCD) technology, light-emitting diode (LED) technology (including, for example, OLED, ILED, µLED, AMOLED, TOLED, etc.), light emitting polymer display (LPD) technology, or some other display technology.

Input/output user interface 1660 may allow a user to send action requests to computing system 1600 to perform particular actions, and may provide information (e.g., status of computing system 1600, measurement results, alerts, etc.) to the user. Input/output user interface 1660 may include one or more input devices, such as, for example, a touchscreen, a touch pad, microphone(s), button(s), dial(s), switch(es), a keyboard, a mouse, a game controller, or any other suitable device for receiving action requests and communicating the received action requests to processor(s) 1610. In some embodiments, input/output user interface 1660 may include one or more output devices, such as a display, a speaker, a light emitting device, a haptic device, and the like, to provide feedback or alarm to the user.

In some embodiments, computing system 1600 may include a plurality of other hardware modules 1670. Each of other hardware modules 1670 may be a physical module within computing system 1600. While each of other hardware modules 1670 may be permanently configured as a structure, some of other hardware modules 1670 may be temporarily configured to perform specific functions or temporarily activated. Examples of other hardware modules 1670 may include, for example, an audio output and/or input module (e.g., a microphone or speaker), a near field communication (NFC) module, a rechargeable battery, a battery management system, a wired/wireless battery charging system, an actuator controller, and the like. In some embodiments, one or more functions of other hardware modules 1670 may be implemented in software.

In one example, computing system 1600 may be part of an insulin delivery device (e.g., a pump) that can deliver fast-acting insulin through a small tube configured for fluidic connection with a cannula inserted subcutaneously. Computing system 1600 may cause the delivery of two types of dosages-a basal dosage, which can be delivered periodically (e.g., every five minutes) in tiny amounts throughout the day and night, and a bolus dosage to cover an increase in blood glucose from meals and/or to correct high blood glucose levels. The insulin delivery device may include a user interface having button elements that can be manipulated to administer a bolus of insulin, to change therapy settings, to change user preferences, to select display features, and the like. The insulin delivery device may also include a display device that can be used to present various types of information or data to the user. In accordance with aspects of the present disclosure, a user of the insulin delivery device may use the button elements to input certain event data (e.g., event type, event start time, event details, etc.), and the user inputs can be confirmed using the display device.

In various implementations, the above-described hardware and modules may be implemented on a single device or on multiple devices that can communicate with one another using wired or wireless connections. In alternative configurations, different and/or additional components may be included in computing system 1600. Similarly, functionality of one or more of the components can be distributed among the components in a manner different from the manner described above.

Embodiments of the methods disclosed herein may be implemented by hardware, software, firmware, middleware, microcode, hardware description languages, or any combination thereof. For example, the operations may be performed by one or more servers or other computer systems. When implemented in software, firmware, middleware, or microcode, the program code or code segments to perform the associated tasks may be stored in one or more computer-readable media such as a storage medium, and may be executed by one or more processors to perform the associated tasks. The computer-readable media may include transitory or non-transitory computer-readable media, such as RAM, ROM, EEPROM, flash memory, solid-state drive, hard drive, CD, DVD, or any other medium that can be used to store desired program code in the form of instructions or data structures and that can be accessed by a computer. The one or more processors may include general purpose microprocessors, application specific integrated circuits (ASICs), graphic processing units (GPUs), network processing units (NPUs), digital signal processors (DSPs), field programmable logic arrays (FPGAs), and the like.

The methods, systems, and devices discussed above are examples. Various embodiments may omit, substitute, or add various procedures or components as appropriate. For instance, in alternative configurations, the methods described may be performed in an order different from that described, and/or various stages may be added, omitted, and/or combined. Also, features described with respect to certain embodiments may be combined in various other embodiments. Different aspects and elements of the embodiments may be combined in a similar manner. Also, technology evolves and, thus, many of the elements are examples that do not limit the scope of the disclosure to those specific examples.

Specific details are given in the description to provide a thorough understanding of the embodiments. However, embodiments may be practiced without these specific details. For example, well-known circuits, processes, systems, structures, and techniques have been shown without unnecessary detail in order to avoid obscuring the embodiments. This description provides example embodiments only, and is not intended to limit the scope, applicability, or configuration of the invention. Rather, the preceding description of the embodiments will provide those skilled in the art with an enabling description for implementing various embodiments. Various changes may be made in the function and arrangement of elements without departing from the spirit and scope of the present disclosure.

Also, some embodiments were described as processes depicted as flow diagrams or block diagrams. Although each may describe the operations as a sequential process, many of the operations may be performed in parallel or concurrently. In addition, the order of the operations may be rearranged. A process may have additional steps not included in the figure. Furthermore, embodiments of the methods may be implemented by hardware, software, firmware, middleware, microcode, hardware description languages, or any combination thereof. When implemented in software, firmware, middleware, or microcode, the program code or code segments to perform the associated tasks may be stored in a computer-readable medium such as a storage medium. Processors may perform the associated tasks.

It will be apparent to those skilled in the art that substantial variations may be made in accordance with specific requirements. For example, customized or special-purpose hardware might also be used, and/or particular elements might be implemented in hardware, software (including portable software, such as applets, etc.), or both. Further, connection to other computing devices such as network input/output devices may be employed.

Any of the herein described techniques, operations, methods, programs, algorithms, or codes may be converted to, or expressed in, a programming language or computer program embodied on a computer, processor, or machine-readable medium. The terms "programming language" and "computer program," as used herein, each include any language used to specify instructions to a computer or processor, and include (but is not limited to) the following languages and their derivatives: Assembler, Basic, Batch files, BCPL, C, C+, C++, Delphi, Fortran, Java, JavaScript, machine code, operating system command languages, Pascal, Perl, PL1, Python, scripting languages, Visual Basic, metalanguages which themselves specify programs, and all first, second, third, fourth, fifth, or further generation computer languages. Also included are database and other data schemas, and any other meta-languages. No distinction is made between languages which are interpreted, compiled, or use both compiled and interpreted approaches. No distinction is made between compiled and source versions of a program. Thus, reference to a program, where the programming language could exist in more than one state (such as source, compiled, object, or linked) is a reference to any and all such states. Reference to a program may encompass the actual instructions and/or the intent of those instructions.

With reference to the appended figures, components that can include memory can include non-transitory machine-readable media. The term "machine-readable medium" and "computer-readable medium" may refer to any storage medium that participates in providing data that causes a machine to operate in a specific fashion. In embodiments provided hereinabove, various machine-readable media might be involved in providing instructions/code to processing units and/or other device(s) for execution. Additionally or alternatively, the machine-readable media might be used to store and/or carry such instructions/code. In many implementations, a computer-readable medium is a physical and/or tangible storage medium. Such a medium may take many forms, including, but not limited to, non-volatile media, volatile media, and transmission media. Common forms of computer-readable media include, for example, magnetic and/or optical media such as compact disk (CD) or digital versatile disk (DVD), punch cards, paper tape, any other physical medium with patterns of holes, a RAM, a programmable read-only memory (PROM), an erasable programmable read-only memory (EPROM), a FLASH-EPROM, any other memory chip or cartridge, a carrier wave as described hereinafter, or any other medium from which a computer can read instructions and/or code. A computer program product may include code and/or machine-executable instructions that may represent a procedure, a function, a subprogram, a program, a routine, an application (App), a subroutine, a module, a software package, a class, or any combination of instructions, data structures, or program statements.

Those of skill in the art will appreciate that information and signals used to communicate the messages described herein may be represented using any of a variety of different technologies and techniques. For example, data, instructions, commands, information, signals, bits, symbols, and chips that may be referenced throughout the above description may be represented by voltages, currents, electromagnetic waves, magnetic fields or particles, optical fields or particles, or any combination thereof.

Terms, "and" and "or" as used herein, may include a variety of meanings that are also expected to depend at least in part upon the context in which such terms are used. Typically, "or" if used to associate a list, such as A, B, or C, is intended to mean A, B, and C, here used in the inclusive sense, as well as A, B, or C, here used in the exclusive sense. In addition, the term "one or more" as used herein may be used to describe any feature, structure, or characteristic in the singular or may be used to describe some combination of features, structures, or characteristics. However, it should be noted that this is merely an illustrative example and claimed subject matter is not limited to this example. Furthermore, the term "at least one of" if used to associate a list, such as A, B, or C, can be interpreted to mean A, B, C, or any combination of A, B, and/or C, such as AB, AC, BC, AA, ABC, AAB, AABBCCC, etc.

Further, while certain embodiments have been described using a particular combination of hardware and software, it should be recognized that other combinations of hardware and software are also possible. Certain embodiments may be implemented only in hardware, or only in software, or using combinations thereof. In one example, software may be implemented with a computer program product containing computer program code or instructions executable by one or more processors for performing any or all of the steps, operations, or processes described in this disclosure, where the computer program may be stored on a non-transitory computer readable medium. The various processes described herein can be implemented on the same processor or different processors in any combination.

Where devices, systems, components or modules are described as being configured to perform certain operations or functions, such configuration can be accomplished, for example, by designing electronic circuits to perform the operation, by programming programmable electronic circuits (such as microprocessors) to perform the operation such as by executing computer instructions or code, or processors or cores programmed to execute code or instructions stored on a non-transitory memory medium, or any combination thereof. Processes can communicate using a variety of techniques, including, but not limited to, conventional techniques for inter-process communications, and different pairs of processes may use different techniques, or the same pair of processes may use different techniques at different times.

The specification and drawings are, accordingly, to be regarded in an illustrative rather than a restrictive sense. It will, however, be evident that additions, subtractions, deletions, and other modifications and changes may be made thereunto without departing from the broader spirit and scope as set forth in the claims. Thus, although specific embodiments have been described, these are not intended to be limiting. Various modifications and equivalents are within the scope of the following claims.

In view of this description embodiments may include different combinations of features. Implementation examples are described in the following numbered clauses:

**Clause 1.** A processor-implemented method comprising:

receiving an input of a target value of a first continuous glucose monitoring (CGM) metric;
estimating a target value of at least a second CGM metric that corresponds to the target value of the first CGM metric; and
providing the estimated target value of at least the second CGM metric to a user.

**Clause 2.** The processor-implemented method of Clause 1, wherein the first CGM metric includes:

glucose management indicator (GMI);
percentage of time of a glucose level in a range of 70-180 mg/dL (TIR);
percentage of time of the glucose level in a range of 70-140 mg/dL (TITR);
percentage of time of the glucose level above 180 mg/dL (TA180);
percentage of time of the glucose level above 250 mg/dL (TA250);
percentage of time of the glucose level below 70 mg/dL (TB70); or
percentage of time of the glucose level below 54 mg/dL (TB54).

**Clause 3.** The processor-implemented method of Clause 1 or 2, wherein the second CGM metric includes GMI, TIR, TITR, TA180, TA250, TB70, or TB54.

**Clause 4.** 4The processor-implemented method of any of Clauses 1-3, wherein estimating the target value of at least the second CGM metric that corresponds to the target value of the first CGM metric comprises:

classifying, using a classifier, CGM data samples meeting and not meeting the target value of the first CGM metric into samples meeting or not meeting each threshold target of a plurality of threshold targets of the second CGM metric;
determining, for each threshold target of the plurality of threshold targets of the second CGM metric, a true positive rate (TPR) and a false positive rate (FPR) of the classifier; and
identifying the target value of at least the second CGM metric based on the TPR and the FPR of the classifier for each threshold target of the plurality of threshold targets of the second CGM metric.

**Clause 5.** The processor-implemented method of Clause 4, wherein identifying the target value of at least the second CGM metric based on the TPR and the FPR of the classifier for each threshold target of the plurality of threshold targets of the second CGM metric comprises:

determining a receiver operating characteristic (ROC) curve of the classifier based on the TPR and the FPR of the classifier for each threshold target of the plurality of threshold targets of the second CGM metric;
selecting a first data point of the ROC curve that is closest to a point having a TPR of one and an FPR of zero; and
identifying a threshold target associated with the first data point as the target value of the second CGM metric that corresponds to the target value of the first CGM metric.

**Clause 6.** The processor-implemented method of Clause 4 or 5, wherein the CGM data samples include at least one of:

CGM data samples of the user;
CGM data samples of a population;
CGM data samples of users in an age group;
CGM data samples of users of an automatic insulin delivery system;
CGM data samples of users living with Type 1 diabetes and/or users living with Type 2 diabetes; or
training samples and test samples.

**Clause 7.** The processor-implemented method of any of Clauses 4-6, wherein the classifier includes a binary classifier.

**Clause 8.** The processor-implemented method of any of Clauses 1-7, further comprising:

determining that the estimated target value of at least the second CGM metric meets a predetermined criterion; and
configuring an insulin delivery system based on the target value of the first CGM metric.

**Clause 9.** A processor-implemented method comprising:

classifying, using a classifier, continuous glucose monitoring (CGM) data samples meeting and not meeting a target value of a first CGM metric as samples meeting or not meeting each threshold target of a plurality of threshold targets of a second CGM metric;
determining, for each threshold target of the plurality of threshold targets of the second CGM metric, a true positive rate (TPR) and a false positive rate (FPR) of the classifier; and
identifying a target value of at least the second CGM metric based on the TPR and the FPR of the classifier for each threshold target of the plurality of threshold targets of the second CGM metric.

**Clause 10.** The processor-implemented method of Clause 9, wherein identifying the target value of at least the second CGM metric based on the TPR and the FPR of the classifier for each threshold target of the plurality of threshold targets of the second CGM metric comprises:

determining a receiver operating characteristic (ROC) curve of the classifier based on the TPR and the FPR of the classifier for each threshold target of the plurality of threshold targets of the second CGM metric;
selecting a first data point of the ROC curve that is closest to a point having a TPR of one and an FPR of zero; and
identifying a threshold target associated with the first data point as the target value of the second CGM metric that corresponds to the target value of the first CGM metric.

**Clause 11.** The processor-implemented method of Clause 9 or 10, wherein the first CGM metric includes:

glucose management indicator (GMI);
percentage of time of a glucose level in a range of 70-180 mg/dL (TIR);
percentage of time of the glucose level in a range of 70-140 mg/dL (TITR);
percentage of time of the glucose level above 180 mg/dL (TA180);
percentage of time of the glucose level above 250 mg/dL (TA250);
percentage of time of the glucose level below 70 mg/dL (TB70); or
percentage of time of the glucose level below 54 mg/dL (TB54).

**Clause 12.** The processor-implemented method of any of Clauses 9-11, wherein the second CGM metric includes GMI, TIR, TITR, TA180, TA250, TB70, or TB54.

**Clause 13.** The processor-implemented method of any of Clauses 9-12, wherein the CGM data samples include at least one of:

CGM data samples of a user;
CGM data samples of a population;
CGM data samples of users in an age group;
CGM data samples of users of an automatic insulin delivery system; or
CGM data samples of users living with Type 1 diabetes and/or users living with Type 2 diabetes.

**Clause 14.** The processor-implemented method of any of Clauses 9-13, wherein the CGM data samples include CGM data samples of users of an automatic insulin delivery system.

**Clause 15.** A system comprising:

one or more processors;
one or more processor-readable storage media storing instructions which, when executed by the one or more processors, cause operations including:

receiving an input of a target value of a first continuous glucose monitoring (CGM) metric;
estimating a target value of at least a second CGM metric that corresponds to the target value of the first CGM metric; and
providing the estimated target value of at least the second CGM metric to a user.

**Clause 16.** The system of Clause 15, wherein estimating the target value of at least the second CGM metric that corresponds to the target value of the first CGM metric comprises:

classifying, using a classifier, CGM data samples meeting and not meeting the target value of the first CGM metric into samples meeting or not meeting each threshold target of a plurality of threshold targets of the second CGM metric;
determining, for each threshold target of the plurality of threshold targets of the second CGM metric, a true positive rate (TPR) and a false positive rate (FPR) of the classifier; and
identifying the target value of at least the second CGM metric based on the TPR and the FPR of the classifier for each threshold target of the plurality of threshold targets of the second CGM metric.

**Clause 17.** The system of Clause 16, wherein identifying the target value of at least the second CGM metric based on the TPR and the FPR of the classifier for each threshold target of the plurality of threshold targets of the second CGM metric comprises:

determining a receiver operating characteristic (ROC) curve of the classifier based on the TPR and the FPR of the classifier for each threshold target of the plurality of threshold targets of the second CGM metric;
selecting a first data point of the ROC curve that is closest to a point having a TPR of one and an FPR of zero; and
identifying a threshold target associated with the first data point as the target value of the second CGM metric that corresponds to the target value of the first CGM metric.

**Clause 18.** The system of any of Clauses 15-17, wherein the first CGM metric includes:

glucose management indicator (GMI);
percentage of time of a glucose level in a range of 70-180 mg/dL (TIR);
percentage of time of the glucose level in a range of 70-140 mg/dL (TITR);
percentage of time of the glucose level above 180 mg/dL (TA180);
percentage of time of the glucose level above 250 mg/dL (TA250);
percentage of time of the glucose level below 70 mg/dL (TB70); or
percentage of time of the glucose level below 54 mg/dL (TB54).

**Clause 19.** The system of any of Clauses 15-18, wherein the second CGM metric includes GMI, TIR, TITR, TA180, TA250, TB70, or TB54.

**Clause 20.** The system of any of Clauses 15-19, wherein the operations further include:

determining if the estimated target value of at least the second CGM metric meets a predetermined criterion; and

configuring an insulin delivery system based on the target value of the first CGM metric.

**Claims**

1. A processor-implemented method comprising:

   receiving an input of a target value of a first continuous glucose monitoring (CGM) metric;
   estimating a target value of at least a second CGM metric that corresponds to the target value of the first CGM metric; and
   providing the estimated target value of at least the second CGM metric to a user.

2. The processor-implemented method of claim 1, wherein the first CGM metric includes:

   glucose management indicator (GMI);
   percentage of time of a glucose level in a range of 70-180 mg/dL (TIR);
   percentage of time of the glucose level in a range of 70-140 mg/dL (TITR);
   percentage of time of the glucose level above 180 mg/dL (TA180);
   percentage of time of the glucose level above 250 mg/dL (TA250);
   percentage of time of the glucose level below 70 mg/dL (TB70); or
   percentage of time of the glucose level below 54 mg/dL (TB54).

3. The processor-implemented method of claim 1 or 2, wherein the second CGM metric includes GMI, TIR, TITR, TA180, TA250, TB70, or TB54.

4. The processor-implemented method of any of claims 1-3, wherein estimating the target value of at least the second CGM metric that corresponds to the target value of the first CGM metric comprises:

   classifying, using a classifier, CGM data samples meeting and not meeting the target value of the first CGM metric into samples meeting or not meeting each threshold target of a plurality of threshold targets of the second CGM metric;
   determining, for each threshold target of the plurality of threshold targets of the second CGM metric, a true positive rate (TPR) and a false positive rate (FPR) of the classifier; and
   identifying the target value of at least the second CGM metric based on the TPR and the FPR of the classifier for each threshold target of the plurality of threshold targets of the second CGM metric.

5. The processor-implemented method of claim 4, wherein identifying the target value of at least the second CGM metric based on the TPR and the FPR of the classifier for each threshold target of the plurality of threshold targets of the second CGM metric comprises:

   determining a receiver operating characteristic (ROC) curve of the classifier based on the TPR and the FPR of the classifier for each threshold target of the plurality of threshold targets of the second CGM metric;
   selecting a first data point of the ROC curve that is closest to a point having a TPR of one and an FPR of zero; and
   identifying a threshold target associated with the first data point as the target value of the second CGM metric that corresponds to the target value of the first CGM metric.

6. The processor-implemented method of claim 4 or 5, wherein the CGM data samples include at least one of:

   CGM data samples of the user;
   CGM data samples of a population;
   CGM data samples of users in an age group;
   CGM data samples of users of an automatic insulin delivery system;
   CGM data samples of users living with Type 1 diabetes and/or users living with Type 2 diabetes; or
   training samples and test samples.

7. The processor-implemented method of any of claims 4-6, wherein the classifier includes a binary classifier.

8. The processor-implemented method of any of claims 1-7, further comprising:

   determining that the estimated target value of at least the second CGM metric meets a predetermined criterion; and
   configuring an insulin delivery system based on the target value of the first CGM metric.

9. A processor-implemented method comprising:

   classifying, using a classifier, continuous glucose monitoring (CGM) data samples meeting and not meeting a target value of a first CGM metric as samples meeting or not meeting each threshold target of a plurality of threshold targets of a second CGM metric;
   determining, for each threshold target of the plurality of threshold targets of the second CGM metric, a true positive rate (TPR) and a false positive rate (FPR) of the classifier; and
   identifying a target value of at least the second CGM metric based on the TPR and the FPR of the classifier for each threshold target of the plurality of threshold targets of the second CGM metric.

10. The processor-implemented method of claim 9, wherein identifying the target value of at least the second CGM metric based on the TPR and the FPR of the classifier for each threshold target of the plurality of threshold targets of the second CGM metric comprises:

    determining a receiver operating characteristic (ROC) curve of the classifier based on the TPR and the FPR of the classifier for each threshold target of the plurality of threshold targets of the second CGM metric;
    selecting a first data point of the ROC curve that is closest to a point having a TPR of one and an FPR of zero; and
    identifying a threshold target associated with the first data point as the target value of the second CGM metric that corresponds to the target value of the first CGM metric.

11. The processor-implemented method of claim 9 or 10, wherein the first CGM metric includes:

    glucose management indicator (GMI);
    percentage of time of a glucose level in a range of 70-180 mg/dL (TIR);
    percentage of time of the glucose level in a range of 70-140 mg/dL (TITR);
    percentage of time of the glucose level above 180 mg/dL (TA180);
    percentage of time of the glucose level above 250 mg/dL (TA250);
    percentage of time of the glucose level below 70 mg/dL (TB70); or
    percentage of time of the glucose level below 54 mg/dL (TB54).

12. The processor-implemented method of any of claims 9-11, wherein the second CGM metric includes GMI, TIR, TITR, TA180, TA250, TB70, or TB54.

13. The processor-implemented method of any of claims 9-12, wherein the CGM data samples include at least one of:

    CGM data samples of a user;
    CGM data samples of a population;
    CGM data samples of users in an age group;
    CGM data samples of users of an automatic insulin delivery system; or
    CGM data samples of users living with Type 1 diabetes and/or users living with Type 2 diabetes.

14. The processor-implemented method of any of claims 9-13, wherein the CGM data samples include CGM data samples of users of an automatic insulin delivery system.

15. A system comprising:

    one or more processors;
    one or more processor-readable storage media storing instructions which, when executed by the one or more processors, cause operations including:

       receiving an input of a target value of a first continuous glucose monitoring (CGM) metric;
       estimating a target value of at least a second CGM metric that corresponds to the target value of the first CGM metric; and
providing the estimated target value of at least the second CGM metric to a user.

**FIG. 1**

**FIG. 2**

**FIG. 3**

EP 4 651 146 A1

400

420
414
430
412
440
412
410

**FIG. 4A**

400

420

416
414
412
418
410
440

**FIG. 4B**

**FIG. 5**

**FIG. 6A**          **FIG. 6B**          **FIG. 6C**

>250 mg/dL
(13.9 mmol/L)

**Target**
**<5%**

>180 mg/dL
(10.0 mmol/L)

**<25%***

Target Range:
70-180 mg/dL
(3.9-10.0 mmol/L)

**>70%**

<70 mg/dL (3.9 mmol/L)
<54 mg/dL (3.0 mmol/L)

**<4%****
**<1%**

*FIG. 7*

| | ≤15 years | | >15 years | |
|---|---|---|---|---|
| | Overall | Optimal Settings | Overall | Optimal Settings |
| Users, n | 14,459 | 1,036 | 41,692 | 3,015 |
| Time in AHCL, % | 90.7 ± 15.0 | 95.2 ± 8.6 | 89.8 ± 16.0 | 93.7 ± 11.2 |
| Mean SG, mg/dL | 151.7 ± 16.1 | 141.6 ± 12.2 | 150.2 ± 16.6 | 140.0 ± 11.6 |
| Standard Deviation of SG, mg/dL | 56.8 ± 11.0 | 50.8 ± 9.2 | 50.5 ± 10.1 | 45.8 ± 8.4 |
| Percentage of users achieving targets: | | | | |
| GMI<7% | 61.3 | 86.8 | 64.6 | 89.2 |
| TIR>70% | 56.6 | 83.5 | 68.3 | 90.2 |
| TITR>50% | 44.9 | 73.9 | 45.9 | 76.4 |
| TB70<4% | 78.9 | 75.4 | 88.9 | 87.3 |
| TB54<1% | 82.9 | 83.5 | 89.6 | 89.8 |
| TA180<25% | 47.3 | 74.8 | 57.0 | 82.2 |
| TA250<5% | 41.8 | 68.3 | 60.7 | 83.0 |

Time in ranges, %

Legend: 54  70  140  180  250  mg/dL ; 3.0  3.9  7.8  10.0  13.9  mmol/L

| Time in ranges, % (top → bottom) | ≤15 Overall | ≤15 Optimal Settings | >15 Overall | >15 Optimal Settings |
|---|---|---|---|---|
| >250 | 7.1 | 4.2 | 5.3 | 3.0 |
| 180–250 | 19.2 | 15.9 | 18.9 | 14.8 |
| 140–180 | 22.0 | 21.6 | 24.9 | 23.6 |
| 70–140 | 48.9 | 55.4 | 48.9 | 56.4 |
| 54–70 | 2.2 | 2.3 | 1.6 | 1.8 |
| <54 | 0.6 | 0.6 | 0.4 | 0.4 |

*FIG. 8*

EP 4 651 146 A1

| | ≤15 years | | >15 years | |
|---|---|---|---|---|
| | Post-AHCL | | Post-AHCL | |
| | Overall | Optimal Settings | Overall | Optimal Settings |
| Users, n | 14,459 | 1,036 | 41,692 | 3,015 |
| Time in AHCL, % | 90.7 ± 15.0 | 95.2 ± 8.6 | 89.8 ± 16.0 | 93.7 ± 11.2 |
| Mean SG, mg/dL | 151.7 ± 16.1 | 141.6 ± 12.2 | 150.2 ± 16.6 | 140.0 ± 11.6 |
| Standard Deviation of SG, mg/dL | 56.8 ± 11.0 | 50.8 ± 9.2 | 50.5 ± 10.1 | 45.8 ± 8.4 |
| Mean GMI, % | 6.9 ± 0.4 | 6.7 ± 0.3 | 6.9 ± 0.4 | 6.7 ± 0.3 |

Time in ranges, %

54   70      180   250    mg/dL

3.0   3.9   10.0   13.9   mmol/L

| | ≤15 years Overall | ≤15 years Optimal Settings | >15 years Overall | >15 years Optimal Settings |
|---|---|---|---|---|
| | 7.1 | 4.2 | 5.3 | 3.0 |
| | 19.2 | 15.9 | 18.9 | 14.8 |
| | 70.9 | 76.9 | 73.8 | 80.0 |
| | 2.2 | 2.3 | 1.6 | 1.8 |
| | 0.6 | 0.6 | 0.4 | 0.4 |

*FIG. 9*

**FIG. 10**

1100

| | 15 years or younger | | | | Older than 15 years | | | |
|---|---|---|---|---|---|---|---|---|
| | GMI<7.0% | GMI<6.8% | GMI<6.6% | GMI<6.5% | GMI<7.0% | GMI<6.8% | GMI<6.6% | GMI<6.5% |
| | TITR 70-140 mg/dL | | | | TITR 70-140 mg/dL | | | |
| Optimal target | >46.9% (46.7, 47.1) | >50.9% (50.7, 51.3) | >55.1% (54.8, 55.7) | >57.1% (56.5, 57.7) | >45.7% (45.4, 45.9) | >50.7% (50.5, 50.8) | >56% (55.7, 56.3) | >58.4% (58.3, 58.8) |
| Sensitivity | 0.91 (0.9, 0.92) | 0.92 (0.91, 0.93) | 0.95 (0.93, 0.96) | 0.95 (0.92, 0.97) | 0.93 (0.93, 0.94) | 0.94 (0.93, 0.95) | 0.95 (0.94, 0.95) | 0.95 (0.93, 0.96) |
| Specificity | 0.92 (0.91, 0.93) | 0.9 (0.89, 0.91) | 0.91 (0.9, 0.92) | 0.92 (0.91, 0.93) | 0.94 (0.93, 0.94) | 0.93 (0.93, 0.94) | 0.93 (0.93, 0.94) | 0.93 (0.92, 0.93) |
| Accuracy | 0.91 (0.9, 0.92) | 0.91 (0.9, 0.92) | 0.92 (0.91, 0.93) | 0.92 (0.91, 0.93) | 0.93 (0.93, 0.94) | 0.94 (0.93, 0.94) | 0.94 (0.93, 0.94) | 0.93 (0.92, 0.93) |
| AUC | 0.98 | 0.98 | 0.98 | 0.98 | 0.98 | 0.99 | 0.99 | 0.99 |
| | TIR 70-180 mg/dL | | | | TIR 70-180 mg/dL | | | |
| Optimal target | >69.6% (69.3, 69.9) | >73.2% (73.1, 73.7) | >76.7% (76.1, 77.1) | >77.7% (77.4, 78.7) | >71.8% (71.8, 72) | >76.1% (76, 76.4) | >79.9% (79.6, 80.1) | >81% (80.8, 81.3) |
| Sensitivity | 0.9 (0.89, 0.91) | 0.89 (0.87, 0.9) | 0.86 (0.83, 0.88) | 0.91 (0.88, 0.93) | 0.92 (0.91, 0.92) | 0.89 (0.89, 0.9) | 0.87 (0.86, 0.88) | 0.88 (0.86, 0.89) |
| Specificity | 0.91 (0.9, 0.92) | 0.86 (0.84, 0.87) | 0.87 (0.86, 0.88) | 0.85 (0.84, 0.86) | 0.92 (0.92, 0.93) | 0.88 (0.87, 0.89) | 0.86 (0.86, 0.87) | 0.84 (0.84, 0.85) |
| Accuracy | 0.91 (0.9, 0.91) | 0.87 (0.86, 0.88) | 0.87 (0.86, 0.88) | 0.86 (0.85, 0.87) | 0.92 (0.91, 0.92) | 0.89 (0.88, 0.89) | 0.86 (0.86, 0.87) | 0.85 (0.84, 0.85) |
| AUC | 0.97 | 0.95 | 0.94 | 0.94 | 0.98 | 0.96 | 0.95 | 0.94 |
| | TA180 mg/dL | | | | TA180 mg/dL | | | |
| Optimal target | <28.1% (27.8, 28.2) | <23.3% (23.2, 23.4) | <19.1% (18.7, 19.3) | <17.4% (16.7, 17.5) | <26.7% (26.5, 26.8) | <21.5% (21.5, 21.8) | <16.9% (16.8, 17.2) | <15% (14.7, 15.2) |
| Sensitivity | 0.95 (0.94, 0.96) | 0.94 (0.93, 0.95) | 0.94 (0.92, 0.96) | 0.98 (0.96, 0.99) | 0.95 (0.94, 0.95) | 0.93 (0.93, 0.94) | 0.94 (0.93, 0.94) | 0.95 (0.94, 0.96) |
| Specificity | 0.95 (0.94, 0.96) | 0.94 (0.93, 0.95) | 0.93 (0.92, 0.94) | 0.92 (0.92, 0.93) | 0.95 (0.94, 0.95) | 0.93 (0.93, 0.94) | 0.92 (0.92, 0.93) | 0.92 (0.92, 0.93) |
| Accuracy | 0.95 (0.94, 0.96) | 0.94 (0.93, 0.95) | 0.93 (0.92, 0.94) | 0.93 (0.92, 0.94) | 0.95 (0.94, 0.95) | 0.93 (0.93, 0.94) | 0.93 (0.92, 0.93) | 0.92 (0.92, 0.93) |
| AUC | 0.99 | 0.98 | 0.98 | 0.99 | 0.99 | 0.99 | 0.98 | 0.98 |
| | TA250 mg/dL | | | | TA250 mg/dL | | | |
| Optimal target | <6.9% (6.8 - 6.9) | <5.1% (4.9 - 5.3) | <3.5% (3.4 - 3.7) | <2.8% (2.8 - 3) | <5% (4.9 - 5.2) | <3.5% (3.4 - 3.7) | <2.4% (2.3 - 2.5) | <1.9% (1.8 - 2) |
| Sensitivity | 0.88 (0.87 - 0.89) | 0.88 (0.86 - 0.89) | 0.88 (0.86 - 0.9) | 0.9 (0.87 - 0.93) | 0.87 (0.87 - 0.88) | 0.88 (0.87 - 0.88) | 0.87 (0.86 - 0.89) | 0.88 (0.87 - 0.9) |
| Specificity | 0.87 (0.85 - 0.89) | 0.83 (0.82 - 0.84) | 0.86 (0.85 - 0.87) | 0.89 (0.88 - 0.9) | 0.88 (0.87 - 0.89) | 0.84 (0.84 - 0.85) | 0.84 (0.83 - 0.84) | 0.86 (0.85 - 0.86) |
| Accuracy | 0.88 (0.87 - 0.89) | 0.85 (0.84 - 0.86) | 0.87 (0.85 - 0.87) | 0.89 (0.88 - 0.9) | 0.87 (0.87 - 0.88) | 0.86 (0.85 - 0.86) | 0.84 (0.84 - 0.85) | 0.86 (0.85 - 0.87) |
| AUC | 0.95 | 0.94 | 0.94 | 0.95 | 0.95 | 0.94 | 0.94 | 0.94 |
| | TB70 mg/dL | | | | TB70 mg/dL | | | |
| Optimal target | >2.4% (2.1, 2.5) | >2.6% (2.4, 2.7) | >3% (2.7, 3.1) | >3.1% (2.9, 3.3) | >1.5% (1.4, 1.5) | >1.7% (1.6, 1.7) | >2% (2, 2.1) | >2.3% (2.2, 2.3) |
| Sensitivity | 0.59 (0.57, 0.61) | 0.61 (0.59, 0.63) | 0.59 (0.56, 0.63) | 0.61 (0.56, 0.66) | 0.63 (0.61, 0.64) | 0.65 (0.63, 0.66) | 0.66 (0.64, 0.68) | 0.67 (0.64, 0.69) |
| Specificity | 0.66 (0.64, 0.69) | 0.66 (0.64, 0.68) | 0.69 (0.67, 0.7) | 0.69 (0.67, 0.7) | 0.68 (0.67, 0.69) | 0.67 (0.66, 0.68) | 0.71 (0.7, 0.71) | 0.73 (0.72, 0.74) |
| Accuracy | 0.62 (0.6, 0.63) | 0.64 (0.63, 0.66) | 0.67 (0.66, 0.69) | 0.68 (0.67, 0.69) | 0.64 (0.64, 0.65) | 0.66 (0.65, 0.67) | 0.7 (0.69, 0.7) | 0.72 (0.71, 0.73) |
| AUC | 0.69 | 0.7 | 0.71 | 0.71 | 0.71 | 0.72 | 0.74 | 0.76 |
| | TB54 mg/dL | | | | TB54 mg/dL | | | |
| Optimal target | >0.4% (0.4 - 0.5) | >0.4% (0.4 - 0.5) | >0.5% (0.5 - 0.6) | >0.5% (0.5 - 0.6) | >0.2% (0.2 - 0.2) | >0.3% (0.2 - 0.3) | >0.3% (0.3 - 0.3) | >0.4% (0.3 - 0.4) |
| Sensitivity | 0.54 (0.52 - 0.56) | 0.55 (0.52 - 0.57) | 0.5 (0.47 - 0.54) | 0.51 (0.46 - 0.56) | 0.58 (0.57 - 0.59) | 0.59 (0.58 - 0.6) | 0.59 (0.57 - 0.61) | 0.59 (0.56 - 0.61) |
| Specificity | 0.57 (0.55 - 0.6) | 0.59 (0.57 - 0.61) | 0.64 (0.62 - 0.65) | 0.65 (0.63 - 0.66) | 0.61 (0.59 - 0.62) | 0.61 (0.6 - 0.62) | 0.65 (0.64 - 0.66) | 0.66 (0.65 - 0.67) |
| Accuracy | 0.55 (0.54 - 0.57) | 0.57 (0.56 - 0.59) | 0.62 (0.6 - 0.63) | 0.63 (0.62 - 0.65) | 0.59 (0.58 - 0.6) | 0.6 (0.59 - 0.61) | 0.64 (0.63 - 0.64) | 0.65 (0.64 - 0.66) |
| AUC | 0.61 | 0.61 | 0.62 | 0.62 | 0.63 | 0.64 | 0.66 | 0.68 |

*FIG. 11*

EP 4 651 146 A1

FIG. 12A

FIG. 12B

FIG. 12C

FIG. 12D

1300

| 15 years or younger | | | |
|---|---|---|---|
| | TB70 mg/dL <4% | TB70 mg/dL <3.5% | TB70 mg/dL <3% | TB70 mg/dL <2.5% |
| | TB54 mg/dL | | | |
| Optimal target | <0.73% (0.71 - 0.74) | <0.64% (0.61 - 0.65) | <0.55% (0.54 - 0.58) | <0.45% (0.43 - 0.46) |
| Sensitivity | 0.89 (0.88 - 0.9) | 0.89 (0.87 - 0.9) | 0.91 (0.9 - 0.92) | 0.89 (0.88 - 0.9) |
| Specificity | 0.89 (0.87 - 0.91) | 0.89 (0.87 - 0.91) | 0.86 (0.85 - 0.88) | 0.87 (0.85 - 0.88) |
| Accuracy | 0.89 (0.88 - 0.9) | 0.89 (0.88 - 0.9) | 0.89 (0.88 - 0.9) | 0.88 (0.87 - 0.89) |
| AUC | 0.97 | 0.96 | 0.96 | 0.96 |
| Older than 15 years | | | |
| | TB70 mg/dL <4% | TB70 mg/dL <3.5% | TB70 mg/dL <3% | TB70 mg/dL <2.5% |
| | TB54 mg/dL | | | |
| Optimal target | <0.73% (0.68 - 0.73) | <0.61% (0.6 - 0.64) | <0.52% (0.51 - 0.53) | <0.43% (0.4 - 0.43) |
| Sensitivity | 0.92 (0.92 - 0.93) | 0.91 (0.9 - 0.92) | 0.91 (0.91 - 0.92) | 0.91 (0.91 - 0.92) |
| Specificity | 0.92 (0.91 - 0.94) | 0.92 (0.91 - 0.93) | 0.9 (0.89 - 0.91) | 0.89 (0.88 - 0.9) |
| Accuracy | 0.92 (0.92 - 0.93) | 0.91 (0.91 - 0.92) | 0.91 (0.9 - 0.91) | 0.91 (0.9 - 0.91) |
| AUC | 0.98 | 0.98 | 0.97 | 0.97 |

*FIG. 13*

EP 4 651 146 A1

1400

```
                                                              1410
┌──────────────────────────────────────────────────────────────┐
│ Classify, using a classifier, CGM data samples meeting and not │
│ meeting a target value of a first CGM metric as samples meeting │
│ or not meeting each threshold target of a plurality of threshold│
│        targets of a second CGM metric                          │
└──────────────────────────────────────────────────────────────┘
                              │
                              ▼
                                                              1420
┌──────────────────────────────────────────────────────────────┐
│ Determine, for each threshold target of the plurality of       │
│ threshold targets of the second CGM metric, a true positive     │
│ rate (TPR) and a false positive rate (FPR) of the classifier    │
└──────────────────────────────────────────────────────────────┘
                              │
                              ▼
                                                              1430
┌──────────────────────────────────────────────────────────────┐
│ Determine a receiver operating characteristic (ROC) curve of    │
│ the classifier based on the TPR and the FPR of the classifier   │
│ for each threshold target of the plurality of threshold targets │
│            of the second CGM metric                             │
└──────────────────────────────────────────────────────────────┘
                              │
                              ▼
                                                              1440
┌──────────────────────────────────────────────────────────────┐
│ Select a first data point of the ROC curve that is closest to a │
│ point having a TPR of one and an FPR of zero                    │
└──────────────────────────────────────────────────────────────┘
                              │
                              ▼
                                                              1450
┌──────────────────────────────────────────────────────────────┐
│ Identify the threshold target associated with the first data    │
│ point as the target value of the second CGM metric that         │
│ corresponds to the target value of the first CGM metric         │
└──────────────────────────────────────────────────────────────┘
```

*FIG. 14*

1500

```
┌─────────────────────────────────────────────────────────────┐  1510
│                                                               │
│       Receive an input of a target value of a first CGM metric │
│                                                               │
└─────────────────────────────────────────────────────────────┘
                              │
                              ▼
┌─────────────────────────────────────────────────────────────┐  1520
│ Estimate a target value of at least a second CGM metric that  │
│ corresponds to the target value of the first CGM metric        │
└─────────────────────────────────────────────────────────────┘
                              │
                              ▼
┌─────────────────────────────────────────────────────────────┐  1530
│                                                               │
│  Provide the estimated target value of at least the second    │
│  CGM metric to a user                                         │
└─────────────────────────────────────────────────────────────┘
                              │
                              ▼
      No          ◇ Does the estimated target value of at least ◇  1540
  ←───────────────  the second CGM metric meet a criterion?
                              │
                              ▼ Yes
┌─────────────────────────────────────────────────────────────┐  1550
│ Configure an insulin delivery system based on the target      │
│ value of the first CGM metric                                 │
└─────────────────────────────────────────────────────────────┘
```

*FIG. 15*

**FIG. 16**

Europäisches
Patentamt

European
Patent Office

Office européen
des brevets

## EUROPEAN SEARCH REPORT

**Application Number**

EP 25 17 5433

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LU JINGYI ET AL: "Glycemic variability modifies the relationship between time in range and hemoglobin A1c estimated from continuous glucose monitoring: A preliminary study", DIABETES RESEARCH AND CLINICAL PRACTICE, vol. 161, 1 March 2020 (2020-03-01), page 108032, XP093316789, NL ISSN: 0168-8227, DOI: 10.1016/j.diabres.2020.108032 * the whole document * | 1-15 | INV. G16H20/17 A61B5/00 G16H50/20 G16H50/30 G16H50/70 |
| X | US 2022/395200 A1 (KOVATCHEV BORIS P [US] ET AL) 15 December 2022 (2022-12-15) * paragraph [0098] - paragraph [0108] * * paragraph [0145] - paragraph [0160]; figures 1-7 * | 1-15 | |
| A | BERGENSTAL RICHARD M. ET AL: "Glucose Management Indicator (GMI): A New Term for Estimating A1C From Continuous Glucose Monitoring", DIABETES CARE, vol. 41, no. 11, 17 September 2018 (2018-09-17), pages 2275-2280, XP093316644, US ISSN: 0149-5992, DOI: 10.2337/dc18-1581 Retrieved from the Internet: URL:https://diabetesjournals.org/care/article-pdf/41/11/2275/527005/dc181581.pdf> * the whole document * | 1-15 | **TECHNICAL FIELDS SEARCHED (IPC)** G16H A61B |

-/--

The present search report has been drawn up for all claims

| Place of search | Date of completion of the search | Examiner |
|---|---|---|
| Munich | 23 September 2025 | Eichenauer, Lars |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

**Application Number**

EP 25 17 5433

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DAGDELEN SELCUK ET AL: "Expert Panel Recommendations for Use of Standardized Glucose Reporting System Based on Standardized Glucometrics Plus Visual Ambulatory Glucose Profile (AGP) Data in Clinical Practice", FRONTIERS IN ENDOCRINOLOGY, vol. 12, 24 January 2022 (2022-01-24), XP093316883, CH ISSN: 1664-2392, DOI: 10.3389/fendo.2021.663222 * the whole document * | 1-15 | |
| X,P | JAVIER CASTAÑEDA ET AL: "The interdependence of targets for continuous glucose monitoring outcomes in type 1 diabetes with automated insulin delivery", DIABETES, OBESITY AND METABOLISM, BLACKWELL SCIENCE, GB, vol. 26, no. 12, 26 September 2024 (2024-09-26), pages 5836-5844, XP072729337, ISSN: 1462-8902, DOI: 10.1111/DOM.15955 * the whole document * | 1-15 | TECHNICAL FIELDS SEARCHED (IPC) |

The present search report has been drawn up for all claims

| Place of search | Date of completion of the search | Examiner |
|---|---|---|
| Munich | 23 September 2025 | Eichenauer, Lars |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 2 of 2**

## ANNEX TO THE EUROPEAN SEARCH REPORT
## ON EUROPEAN PATENT APPLICATION NO.

EP 25 17 5433

This annex lists the patent family members relating to the patent documents cited in the above-mentioned European search report.
The members are as contained in the European Patent Office EDP file on
The European Patent Office is in no way liable for these particulars which are merely given for the purpose of information.

23-09-2025

| Patent document cited in search report | Publication date | Patent family member(s) | Publication date |
|---|---|---|---|
| US 2022395200 A1 | 15-12-2022 | EP 4057898 A1 | 21-09-2022 |
| | | US 2022395200 A1 | 15-12-2022 |
| | | WO 2021097396 A1 | 20-05-2021 |

EPO FORM P0459

For more details about this annex : see Official Journal of the European Patent Office, No. 12/82

**REFERENCES CITED IN THE DESCRIPTION**

**Patent documents cited in the description**

- US 19488925
- US 63648352